(19) Europäisches Patentamt

European Patent Office

Office européen des brevets

(11) **EP 4 309 625 A1**

(12) **EUROPEAN PATENT APPLICATION**

(43) Date of publication:
**24.01.2024 Bulletin 2024/04**

(21) Application number: **22185918.4**

(22) Date of filing: **20.07.2022**

(51) International Patent Classification (IPC):
***A61F 2/24*** *(2006.01)*

(52) Cooperative Patent Classification (CPC):
**A61F 2/2436;** A61F 2002/9665

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Creganna Unlimited Company
Ballybrit, Galway H91 VN2T (IE)**

(72) Inventors:
• **COONEY, Gerard
  Galway, H91 VN2T (IE)**
• **MCDERMOTT, Bernard
  Galway, H91 VN2T (IE)**

(74) Representative: **Grünecker Patent- und
Rechtsanwälte
PartG mbB
Leopoldstraße 4
80802 München (DE)**

(54) **SOCKET FOR A PROSTHETIC AORTIC VALVE DELIVERY DEVICE, DEVICE FOR DELIVERING A PROSTHETIC AORTIC VALVE, AND METHOD OF CONFIGURING A DEVICE FOR SELECTIVE PROSTHETIC AORTIC VALVE ORIENTATION**

(57) The present disclosure relates to a socket for a prosthetic aortic valve delivery device, rotatable relative to an axis of the delivery device and further relates to a device for delivering a prosthetic aortic valve into an implantation site, and to a method of configuring a device for selective prosthetic aortic valve orientation. A retention socket is provided for retaining a prosthetic aortic valve in a delivery device, the retention socket being configured to be rotatable relative to a longitudinal axis of the delivery device, the socket comprising at least one retention recess positioned on an external surface of the socket for engaging with a paddle or other retention feature of the prosthetic aortic valve.

**Fig. 7**

EP 4 309 625 A1

**Description**

The present disclosure relates to a socket for a prosthetic aortic valve delivery device, rotatable relative to an axis of the delivery device and further relates to a device for delivering a prosthetic aortic valve into an implantation site, and to a method of configuring a device for selective prosthetic aortic valve orientation.

Devices for delivering a prosthetic aortic valve into an implantation site exist. Transcatheter aortic valve implantation, TAVI, or transcatheter aortic valve replacement, TAVR, is a minimally invasive technique for the delivery and deployment of a prosthetic aortic valve or in other words replacement aortic valve or implant. It is used to treat patients with severe aortic stenosis as a less traumatic procedure opposed to more risky traditional open heart surgery or less effective drug therapies. TAVI utilises the body's circulatory system to gain access to the target site, requiring only a small percutaneous incision in most cases. There are three access options with transfemoral being the most commonly used.

Using x-ray and echocardiography to visualise the location of the valve/delivery system within the anatomy and assess blood flow functionality, the physician directs the delivery system to the target location before deploying the replacement valve There are two types of prosthetic aortic valve available in the market: self-expanding frames made with shape memory nickel titanium alloy (NiTi) and balloon expanding frames generally made with pliant cobalt chromium alloy (CoCr).

Self expanding valves are typically shape set to the end shape they will ideally take in-vivo. The super elastic properties of NiTi allow this type of implant to be compressed significantly to a smaller profile in order to make it possible to transport it to the target location within the patients body, whereupon it can be released to take its set shape. In order to keep the implant from expanding before reaching the target location, a thin walled tubular element, in other words capsule, is used to house and retain the implant as it passes through the vasculature. At the target location the capsule is pulled back from the implant to release the valve and allow it to expand and deploy.

A large percentage of TAVI patients have or will develop coronary artery disease. Assuming that TAVR devices last for 10 years or more, the progression of coronary artery disease will eventually require potential diagnostic or therapeutic intervention. Future coronary intervention is a very important consideration as TAVR is expanded to younger patients. Assuming that the replacement valve will degenerate and eventually fail in approximately 10-15 years, it will itself need to be replaced by implanting another replacement valves inside the initial replacement valve.

There is a need for a device to deliver a prosthetic aortic valve i.e. implant, that allows alignment of the prosthetic valve commissures with the native commissures of the patient to avoid obstruction of the coronary arteries by the replacement valve commissures. There is also a need for a method of enabling TAVR commissural alignment with native valve commissures to avoid obstruction of the coronary arteries and for a method of assembling the device. Alignment of the valve commissures is important to avoid blocking the coronary arteries which may prevent future critical coronary interventions. Valve in valve procedures will increase the need for commissure alignment.

In the state of the art delivery devices it is problematic to be able to rotate the implant to achieve alignment prior to deployment. This is problematic to achieve due to the construction of the state of the art TAVI devices.

TAVI delivery devices for self expanding implants are typically able to bend predominantly in a single plane, to allow the device to navigate across the aortic arch and into position inside the native valve. They can also be flexible in multiple planes. High axial stiffness of the inner and outer shaft components is an important requirement to reduce compression and elongation of the shaft during the procedure which would negatively affect positional accuracy and control of the implant deployment. High axial stiffness is also required to facilitate recapture of the implant should this be required. Axial stiffness can be maximised in a number of ways, one being by addition of stiff axial elements on the neutral axis which allow bending of the device in a single primary bending plane but restricts bending out of the primary bending plane. When such a device with a single bending plane is curved in position around the aortic arch to position the implant inside the native valve, it is not possible to rotate the inner or outer shaft around their central axis. This means that rotation of the device, the capsule containing the implant and the implant around its own axis when in a bent configuration to navigate around the aortic arch by means of rotation of the device handle is not possible. The term shaft may also be referred to as sheath.

The present invention is defined by the independent claims. Advantageous embodiments of the present invention are defined by the dependent claims.

The present invention is based on the idea that by providing a rotating implant retention socket element that allows independent rotational positioning of the socket and the implant relative to other parts of the delivery device, alignment of the prosthetic valve's commissures with the native commissures can be achieved. The implant retention socket may be referred as rotatable implant retention socket, prosthetic valve retention socket, retention socket or socket. The prosthetic valve may also be referred to as self expanding prosthetic valve, prosthetic aortic valve or implant.

In the present invention, to allow selective orientation of the implant i.e. prosthetic aortic valve, within the delivery system, the implant retention socket is made independently rotatable to the rest of the system i.e. it can rotate freely around the inner shaft and is constrained from moving axially, either distally or proximally by collars or other features.

By determining the location of the ostia, the native commissures and the orientation of the ascending aorta and arch by pre-procedural imaging, the optimal angular position of the implant within the delivery device referred to as implantation angle, relative to the primary bending plane can be determined to ensure the optimal alignment of the implant commissures with the native valve commissures.

A first method of the invention allows the implant (prosthetic aortic valve) to be orientated during its loading in the delivery system i.e. when being assembled into the delivery system, by means of rotation around its longitudinal axis to optimize the position of the implant commissures or other features with respect to the native commissures and the coronary ostia. Once loaded, the delivery and deployment of the implant is carried out as per normal resulting in optimal alignment of the implant with respect to the coronary ostia.

The first method of the invention is based on arranging a socket rotatable relative to an axis of the delivery device and rotating the socket to an implantation angle relative to a capsule. The implantation angle corresponds to an angle that a primary commissure of the prosthetic aortic valve is configured to make relative to the native coronary arteries. The rotatable socket is rotated and locked at the implantation angle prior to loading the prosthetic valve. At least a first paddle of the prosthetic valve connected to a frame is engaged with a retention recess of the socket so that a rotation of the socket is transmitted to the prosthetic valve. This method is applicable to devices with a single plane of deflection.

A second method of the invention allows additional rotational orientation of the implant during the procedure of delivery and deployment of the implant. The second method of the invention is applicable to devices with bending flexibility in multiple or all planes as opposed to a single plane. The second method allows selective commissure alignment before and/or during in-vivo positioning/deployment of the prosthetic valve that will allow for Percutaneous Coronary Intervention (PCI) access for potential future procedures. A rotatable socket is arranged in the delivery device. Upon loading the prosthetic valve and/or upon inserting the prosthetic valve into a patient, as the section of the handle connected to the outer shaft and capsule is torqued by a clinician relative to a section of the handle connected to the inner shaft, the torque is transferred to the capsule. Through friction, the capsule transfers the torque to the prosthetic valve and to the socket to which the prosthetic valve is secured by means of the at least one paddle of the valve being engaged with the at least one retention recesses of the socket which is free to rotate.

Along with rotating implant retention socket as previously outlined, the device is configured so that the capsule and the outer shaft connected to the capsule can be manually rotated relative to the inner shaft by means of a rotating section of the handle or other such method.

With single plane of deflection devices, the rotational orientation of the capsule and implant relative to the native valve is predictable. Knowing the position of the coronary artery ostia relative to the angle of the aortic arch in the body means that the effective position of the TAVI implant commissures relative to the native coronary arteries can be predicted. The location of the ostia, the native commissures and the orientation of the ascending aorta and arch can be determined by pre-procedural imaging.

Devices which are flexible in multiple planes can also be constrained from device, capsule and implant rotation around their own axis by means of rotating the handle due to the components and assembly's high bending stiffness as the combined bending stiffness of a typical assembly is considerably greater than that which would be required to avoid the distal tip from rotating around the axis of the handle rather than around its own axis.

For both the first and the second methods, as some or all of the implant paddles may not be positioned on the neutral axis of the device as it bends around the aortic arch and vasculature as it is manoeuvred into position to deploy the implant, there is a possibility that there will be additional stresses on the implant struts as the implant conforms to the device geometry and that some struts will be under compressive or other stress.

To compensate for the uneven stresses in bending, one or more of the paddle recesses in the retention socket may be axially elongated so that the implant paddle in these elongated recesses can move axially while still being restrained radially and circumferentially. This movement will assist in relieving the stresses in the implant struts.

The space for the valve paddle to move in the socket on one side of the rotating socket element to help minimize stresses incurred through passive or active bending of the distal catheter shaft/capsule/inner member. This design allows the valve frame to passively respond to external stimulus, whether that external stimulus be applied by the body or the delivery catheter.

In particular, the present invention provides a retention socket for retaining a prosthetic aortic valve in a delivery device, the retention socket being configured to be rotatable relative to an axis of the delivery device, the retention socket comprising: at least one retention recess positioned on an external surface of the socket for engaging with a paddle or other retention feature of the prosthetic aortic valve. The axis of the delivery device may be referred to as a longitudinal axis or a central axis of the delivery device.

Advantageously, prior to loading the prosthetic valve, the retention socket can easily be rotated to a desired orientation angle i.e. implantation angle, relative to an axis of the delivery device such as a catheter shaft's plane of inflexibility, a capsule or relative to any stiff axial element of the catheter. Advantageously, the socket can be fixed at the rotation position corresponding to the later implantation angle at which the commissures of the prosthetic valve do not overlap with the native coronary arteries i. e. at the rotational position at which the commissures of the prosthetic valve are aligned with the native commissures. In other words, it allows commissural alignment between prosthetic and native aortic valve leaflets.

Then the prosthetic valve can be prepped in the loading system and at least one paddle of the prosthetic valve can be placed in the retention recess of the socket remaining fixed at the desired rotation.

Advantageously, the at least one retention recess of the socket is configured to engage with a paddle of the prosthetic valve such that the recess fits and holds the paddle. Thus, the valve paddle can be seated on the retention recess during loading with a special loading tool.

According to an advantageous embodiment, one retention recess fits a paddle of the prosthetic aortic valve so that the paddle is restrained axially, while all other recesses allow some axial movement of the paddles. Advantageously, this configuration reduces stresses in the struts due to bending of the prosthetic valve (implant) inside the bending capsule. The bending causes the strut lengths and paddles to move axially relative to the socket/recesses so if all recesses constrained axial movement then the stresses in the paddles/struts would be higher than if all but one allowed axial movement as in the present invention.

According to an advantageous embodiment, the socket is configured to be independently rotatable to the rest of the system, rotating relative to the inner shaft, the outer shaft and the handle. Advantageously, this facilitates selective orientation of the TAVI within the delivery system. As the socket is rotated into the chosen position, both the capsule and inner shaft, in other words inner catheter or inner shaft, may remain fixed in their orientation. Advantageously, the socket simplifies the rotational mechanism, because it comprises minimal additional components. Additionally, the costs of addition or implementation of this feature is relatively low.

Advantageously, the implant retention socket may require little prosthetic valve changes to reduce strut stress, therefore such a TAVI system will have a shorter development path.

According to an advantageous embodiment the retention recess is configured to engage with a paddle of the prosthetic aortic valve such that upon being engaged the paddle remains detachably locked into the retention recess.

According to an advantageous embodiment at least one of the retention recesses has an axis of symmetry, wherein a length along the axis of the retention recess is larger than the length along the axis of the paddle configured to engage with that recess. Advantageously, the paddle configured to engage with that recess has one degree of freedom of movement along the axis upon the delivery device undergoing a bend.

According to an advantageous embodiment, a length along the axis of at least one retention recesses equals the length along the axis of the paddle configured to engage with the recess. Equals means approximately equal or equal with a small amount of clearance. Advantageously, the paddle configured to engage with that recess cannot move axially.

According to an advantageous embodiment, the socket is configured to be rotatable an implantation angle.

Preferably the socket is rotated the implantation angle relative to a device's plane of inflexibility such as a spine-wire or relative to a handle or a tip of the delivery device. Preferably, the socket is configured to be rotatable by a predetermined implantation angle. Preferably, the implantation angle corresponds to an angle that a primary commissure of the prosthetic aortic valve is configured to make relative to a reference plane defined by a planar angle of an aortic arch to the body.

An advantage deriving from rotating the socket to the implantation angle is that it avoids that the commissures of the prosthetic valve overlap with the coronary artery ostia and allows for the commissures of the prosthetic valve to be aligned, i.e. superposed, with the native commissures or to be close to alignment with the native commissures. Prior to loading the prosthetic valve, the socket can be rotated to the desired implantation angle. When the prosthetic valve is positioned by engaging its paddles with the retention recesses of the socket positioned at the implantation angle, the prosthetic valve remains positioned at the implantation angle that avoids overlap of the prosthetic valve commissures with the coronary arteries.

The retention recess may have more than one recess, the recesses may be of different lengths so that at least one paddle is constrained from axial movement while the other paddle can move axially. This reduces the stresses on the implant struts and paddles once the implant is loaded and the delivery device bends to enable it to navigate across the aortic arch and into position within the native valve.

According to an advantageous embodiment, the implantation angle is determined by defining a right coronary angle, $\alpha_r$, that the right coronary artery of the body makes relative to the reference plane around a circumference of an annulus, determining a left coronary angle, $\alpha_l$, that the left coronary artery of the body makes relative to the reference plane around the circumference of the annulus and determining $$\alpha_{i-anti-clockwise} = 90^o - \left(\left(\frac{\alpha_r+\alpha_l}{2}\right) - 180^o\right)$$ or

$$\alpha_{i\text{-}clockwis} = 270° - \left(\left(\frac{\alpha_r + \alpha_l}{2}\right) - 180°\right)$$ as the implantation angle.

Advantageously this solution ensures that the implantation angle equally bisects right and left coronary ensuring that the commissures of the prosthetic aortic valve, aligned with the implantation angle reference. Thus, the prosthetic valve itself being engaged with the retention recesses of the socket which is rotated to the implantation angle does not overlap with the coronary arteries.

The measurements may be kept relative depending on whether the end up more or less on a negative or positive axis.

According to one advantageous embodiment, the implantation angle may be measured relative to a catheter shaft's plane of inflexibility, the delivery catheter shaft design being such that it only has a significant degree of freedom of movement in one axis. This is advantageous in systems with one plane of deflection as it makes predictive TAVR placement possible.

Axial stiffness of the inner and outer shafts (sheath) components of a delivery catheter may be maximised by addition of stiff axial elements which restrict bending out of the primary bending plane. The position of stiff axial elements define a plane of inflexibility. TAVI devices with a single plane of bending do not allow rotation of the capsule and implant around its own axis by means of rotating the handle. In this configuration, the measuring of the implantation angle relative to the plane of inflexibility and applying it in advance during system setup is carried out by a Therapy Delivery Specialist, TDS, prior to the procedure. This allows for a simple and safe implantation procedure performed a posteriori by the physician that differs little from typical commercial self-expanding TAVI procedures.

A TDS refers to trained professionals, not necessarily a doctor or a physician, that are either employed, trained and provided by a device manufacturer or are separately employed and trained as per the guidelines of the manufacturer. A TDS may not be a doctor and the process of setting up the system carried out by a TDS happens outside and away from a live body.

According to another advantageous embodiment, the implantation angle may be measured, and set, relative to a handle of the delivery catheter either during system setup by a TDS, or during the implant procedure by the physician. This is advantageous in catheters with multiple planes of deflection. The physician can keep the orientation of the system relative by means of ensuring the handle with flush port, or other feature, remains upright while the system is tracked through the iliacs, along the abdominal aorta, around the aortic arch until the capsule is in position across the native annulus. In these systems the implantation angle is measured relative to a handle, a handle flush port or other handle feature rather than a stiff axial element as is the case with single plane deflection devices.

Systems with bending flexibility in all planes can also be constrained from device, capsule and implant rotation around their own axis by means of rotating the handle because the torque transmission from handle to the distal end is generally poor so rotation of the handle does not result in rotation of the distal tip around its own axis. When the distal end is curved around the aortic arch and the handle is rotated, the distal tip tends to rotate around the axis of the handle rather than around its own axis (the axis of the implant). This means that rotational alignment of the implant commissures with the native commissures can be problematic using devices with all plane bending flexibility.

According to an advantageous embodiment, the socket further comprises locking means which lock a rotation of the socket upon being set to the implantation angle. A rotation of the socket refers to a rotational position or orientation. Advantageously the socket can be fixed at the rotation position corresponding to the implantation angle at which the commissures of the prosthetic valve do not overlap with the native coronary arteries. Advantageously the socket rotation can be fixed at the rotation position at which the commissures of the prosthetic valve are aligned (overlap) with the native (natural) commissures. This allows commissural alignment between (bio)prosthetic and native aortic valve leaflets.

According to an advantageous embodiment, the socket further comprises at least a marking to indicate an orientation of the socket relative to a plane of a capsule of the delivery device. According to an advantageous embodiment, the marking indicates an orientation of the socket relative to a plane of anisotropic stiffness of the capsule or a plane of inflexibility. This is advantageous in of devices with a single plane deflection.

According to an advantageous embodiment, the marking indicates an orientation of the socket relative to any plane of the capsule. This is advantageous in devices with multiple planes of deflection.

In other words, in a first design the stiffness is anisotropic and in a second design, the stiffness is isotropic, which means that in both cases there could be different ways to relatively orient as per markings. The markings in the first design would be specific to the plane of inflexibility and the markings in the second design would be specific to any potential orientation that would simplify the procedure and would be fluoro-opaque.

A relative marking on the capsule can indicate the plane of inflexibility in devices with a single plane of deflection, or it can indicate the position of a radiopaque marker on the capsule in devices with multiple plains of deflection which helps the physician observe implant orientation of a fully or partially encapsulated valve during the procedure.

Preferably, the marking may be a 90° cut on the surface of the socket. The marking may be other cuts in the socket at different angles. Preferably the 90° cut or other markings mark the relative orientation of the rotating socket to the capsule. The capsule or the shaft of the delivery catheter may also have a relative marking to provide the orientation of the socket's marking relative to the capsule or shaft.

According to an advantageous embodiment, the socket's marking indicates an orientation of the socket relative to a mark and/or radiopaque marker on a tip or a handle of the delivery device. This is advantageous in devices with multiple planes of deflection.

According to an advantageous embodiment, the socket further comprises an indexing feature to index around a circumference at fixed degrees of rotation. Advantageously the implantation angle can be measured more accurately.

According to an advantageous embodiment, the socket comprises at least a pair of retention recesses, for each pair: the two retention recess are symmetrically positioned on opposite sides of an external surface of the socket; each of the two retention recesses is configured to engage with a paddle of the prosthetic aortic valve such that upon being engaged the paddle remains detachably locked into the retention recess; each retention recess has an axis of symmetry along which a length of the paddle configured to engage with that recess extends; a length along the axis of one of the two recesses is larger than the length along the axis of the paddle configured to engage with that recess and, a length along the axis of the other recess equals the length along the axis of the paddle configured to engage with that other recess so as to allow the paddle configured to engage with the recess of larger length one degree of freedom of movement along the axis, while the paddle configured to engage with the other recess cannot move axially, upon the delivery device undergoing a bend in a plane of deflection associated to that pair of retention recesses which contains the axes of the two retention recesses of that pair.

This embodiment relates to an example of configuration in which there are two retention recesses. However, as described above, there can be a single recess or multiple recesses. The key is that one recess is a good fit to the paddle so the paddle is restrained axially, all other recesses allow some axial movement in order to reduce stresses in the struts due to bending of the implant inside the bending capsule. This bending causes the strut lengths and paddles to move axially relative to the socket/recesses so if all recesses constrained axial movement then the stresses in the paddles/struts would be higher than if all but one allowed axial movement.

The bending of the capsule in turn causes passive bending of the valve, for which the extra space in one valve socket acts to reduce tensile or compressive stresses on the valve socket elements.

According to an advantageous embodiment, a device for delivering a prosthetic aortic valve into an implantation site, comprises a proximal end locatable in proximity to a clinician and a distal end locatable distant from the clinician, the device comprising: a socket according to any of the embodiments disclosed above. Advantageously the device allows to rotate a prosthetic aortic valve assembled in the device, to a fixed orientation angle i.e. implantation angle, at which the commissure i.e. primary commissure, of the prosthetic aortic valve does not overlap with the native commissures. The prosthetic aortic valve delivery device may be referred to as prosthetic aortic valve delivery catheter.

According to an advantageous embodiment, the device further comprises the prosthetic aortic valve comprising at least a first paddle connected to a frame, the at least first paddle being engaged with the at least one retention recess. Advantageously, in this configuration the prosthetic valve is assembled, in other words loaded, in the device. Advantageously the device allows to assemble a prosthetic aortic valve in the device at a fixed implantation angle at which the commissure i.e. primary commissure, of the prosthetic aortic valve does not overlap with the native commissures. The at least first paddle engaged with the at least retention recess may mean that the shape of the paddle fits the shape of the retention recess. The paddle engaged with the retention recess remains detachably locked in the retention recess.

According to an advantageous embodiment one or more of the retention recesses has an axis of symmetry along which a length of the paddle engaged with that recess extends and a length along the axis of the retention recess is larger than the length along the axis of the paddle engaged with that recess. Advantageously this allows the paddle or paddles engaged with this recess one degree of freedom of movement along the axis.

According to an advantageous embodiment one of the retention recesses has an axis of symmetry along which a length of the paddle engaged with that recess extends and a length along the axis of the retention recess equals the length along the axis of the paddle engaged with the recess. Advantageously, the paddle engaged with this recess cannot move axially.

According to an advantageous embodiment at least one of the retention recess is a sliding anchor and the paddle engaged with that recess has a narrow shape of a constant width.

According to an advantageous embodiment, the device further comprises the prosthetic aortic valve comprising a frame and at least a first paddle and a second paddle, wherein the first paddle is engaged with the retention recess of length along the axis larger than the length along the axis of the first paddle and the second paddle is engaged with the retention recess of a length along the axis equal to the length along the axis of the second paddle. This embodiment comprises more than one paddles and socket's retention recess, whereby one or more of the sockets have a longer length to minimize stresses.

An advantage of the larger paddle socket is that thanks to the socket's retention recesses of different lengths, the prosthetic valve is not subjected to stresses when the systems undergoes bending.

According to an advantageous embodiment, the delivery device further comprises an inner shaft having a proximal end connected to the socket.

According to an advantageous embodiment, the delivery device further comprises an outer shaft having at its distal end a capsule that, upon being in a position furthest away from the proximal end of the device, is configured to compress the prosthetic valve within the capsule. Advantageously this avoids movement of the prosthetic aortic valve relative to the capsule and inner shaft and constrains the radial movement of the paddles engaged with the socket.

Advantageously, a capsule of the delivery device can be moved forward to capture the prosthetic valve engaged with the socket, until the tip of the capsule mates with the tip of the inner shaft. Once loaded, the system is passed over the guidewire and into the patient's femoral artery. The system is then tracked through the iliacs, along the abdominal aorta, around the aortic arch until the capsule is in position across the native annulus. When the capsule is pulled back, the prosthetic valve is exposed and then deployed at the desired implantation angle, that is at the desired orientation, across the native annulus allowing for the prosthetic valve commissures to optimally align with the native commissures. Another advantage of this solution is that it allows to position the prosthetic valve in a rotational orientation position with high accuracy.

According to a further advantageous example, the device may comprise an inner shaft having a proximal end connected to the socket and an outer shaft having at its distal end a capsule that, upon being in a position furthest away from the proximal end of the device, is configured to compress the prosthetic valve within the capsule avoiding its relative movement to the capsule and the inner shaft and to constrain the radial movement of the at least one paddles engaged with the socket.

According to an advantageous embodiment the device has one or multiple markings, each marking aligned with a corresponding plane of inflexibility of the delivery device. This is advantageous for systems with a single plane deflection.

According to an advantageous embodiment the device further comprises: a handle connected to the capsule such that when a clinician applies a torque to a section of a handle attached to an outer shaft and to the capsule, relative to a section of the handle attached to an inner shaft, the torque is transferred to the capsule. The capsule is connected to the prosthetic valve by an outward force exerted by the prosthetic valve on the capsule such that the torque is transferred through friction from the capsule to the prosthetic valve and to the socket engaged with the paddles of the prosthetic valve. The socket is configured to freely rotate relative to the inner shaft.

The shaft may also be referred to as sheath. The outer shaft may be directly linked to a capsule outer shaft starting at the exit from the handle and connecting with a distal capsule.

Advantageously the capsule is in contact with the prosthetic valve by an outward force exerted by the prosthetic valve on the capsule such that the torque is transferred through friction from the capsule to the prosthetic valve and to the socket engaged with the paddles of the prosthetic valve. The socket is configured to freely rotate relative to the inner shaft. Advantageously, the torque is transferred through friction from the capsule to the prosthetic valve and to the socket engaged with the paddles of the prosthetic valve.

This In-procedure relative torqueing of the valve according to this embodiment is applicable to devices with multiple planes of deflection. In-procedure relative torqueing of the valve is not possible in devices with single plane of deflection.

Advantageously the implant can rotate freely relative to the inner shaft, given sufficient clearance to the inner shaft or other method of allowing free rotation around the inner shaft. This solution is advantageous to achieve selective commissure alignment before and/or during in-vivo positioning/deployment that will allow for PCI access for potential future procedures. This solution applies to omni-directionally flexible systems. An independently rotatable socket is used to help define the desired orientation of the valve within the body. Along with having the ability to orientate the implant during valve loading, the orientation of the valve can also be adjusted before and during deployment or at any stage while tracking the device through the anatomy.

Advantageously the device is configured so that the capsule, and the outer shaft connected to the capsule, can be manually rotated relative to the inner shaft by means of rotating one part of the handle. As the implant is in a state of compression within the capsule and is applying a radial outward force on the capsule, the implant will tend to rotate with the applied rotation of the capsule. The implant is restrained from axial movement by the retainer. As the retainer is free to rotate around the inner shaft, the implant can also freely rotate around the inner shaft. This allows the implant to be rotated around its own axis in order to align the implant commissures or other features of the implant to best avoid obstruction of the coronary arteries. This rotation of the capsule and implant around its own axis while the delivery device is in place in the aorta/aortic arch and taking its curvature is only possible in a system which has a capsule and outer shaft or other such elements which are free to bend in all directions and do not have a primary or single bend direction.

The inner shaft and elements can be designed to have a single plane of bending or multiple planes. The inner shaft is not required to rotate during the implant rotation, so its bending stiffness does not prevent the outer shaft and capsule from being able to rotate around their own axis. A steering system and/or a method of locking the inner shaft in its deflected position centered in the native valve provides a stable base for capsule and implant rotation around the assembly axis.

According to an advantageous embodiment the device further comprises at least one free floating coil located between the inner sheath component and the implant, positioned on an inner sheath component distal of the implant retention socket with clearance to the inner sheath component, allowing the implant to rotate freely around the inner sheath components upon the capsule and implant being rotated relative to the inner sheath. The clearance may be referred to as extra space between the internal diameter of the socket and the outer diameter of the inner shaft component, if it is too tight, the socket will not freely move. Similarly it cannot be too loose. There needs to be just enough space for free movement where there is no compressive friction. According to an advantageous embodiment, at least one of the valve paddles comprises a sliding anchor (rectangular paddle) configured to slide in the retention recesses with which it is configured to engage. Preferably, the sliding anchor has a shape of constant width. The sliding anchor may be referred to as valve sliding anchor. A valve paddle being a sliding anchor means that it has the shape of a sliding anchor. The sliding anchor may be referred to as a rectangular paddle. Preferably the valve sliding anchor has a constant width. Advantageously, one or more of the sliding anchor paddle has no axial constraint. In other words, the paddle has one degree of freedom of movement along the axis.

According to an advantageous embodiment the retention recess with which a sliding anchor engages has a an elongated shape in which the sliding anchor paddle can slide. Preferably the elongated shape of the retention recess is rectangular. These recesses may engage with a sliding anchor paddle that may be narrow and of a constant width. For example the sliding anchor paddle may be rectangular. These recesses may constrain the implant from rotating relative to the socket i.e. moving circumferentially relative to the socket, but do not constrain the paddle from moving in an axial direction, distally or proximally. Preferably, at least one of the recesses remains at a length which will constrain movement axially both proximally and distally.

According to an advantageous embodiment, a method of configuring a device for selective prosthetic aortic valve orientation comprises the following steps:

- determining a planar angle of an aortic arch of a body to the body, defining a plane;
- determining a right coronary angle, $\alpha_r$, relative to the plane around a circumference of an annulus, determining a left coronary angle, $\alpha_l$, relative the plane around the circumference of the annulus;
- determining an implantation angle, $\alpha_i$, corresponding to an angle that a primary commissure of the prosthetic aortic valve is configured to make relative to the reference plane as

$$\alpha_{i-anti-clockwis} = 90^o - \left(\left(\frac{\alpha_r+\alpha_l}{2}\right) - 180^o\right)$$

or $\alpha_{i-clockwis} = 270^o - \left(\left(\frac{\alpha_r+\alpha_l}{2}\right) - 180^o\right)$;

- arranging a rotatable socket configured to rotate relative to an axis of the delivery device and rotating the socket to the implantation angle relative to a capsule;
- wherein the socket comprises at least one retention recess configured to engage with a paddle of the prosthetic aortic valve, such that that paddle remains detachably locked into the retention recess;
- placing at least one paddle of the prosthetic valve in the at least one retention recess of the socket which is fixed at a rotation corresponding to the implantation angle;
- moving the capsule forward to encapsulate the valve.

The method of configuring a device for selective prosthetic aortic valve orientation may be referred to as method of assembling a device for delivering a prosthetic aortic valve.

If the angle is greater than 180 degrees, it may be difficult for the TDS to set the angle clockwise. In that case it may be easier to measure counter clockwise which would be a smaller angle. Advantageously it is optional for the TDS and MRI/CAT scan technician to choose between clockwise and anti-clockwise.

It should be noted that the actual delivery will be unchanged from how similar devices in the market are used by physicians currently. However, the preparation of the delivery device is novel in view of the calculations that derive from the mmiMRI or x-ray/CAT scan and how the device itself is prepared before being used on a patient. In particular, two fully assembled components, namely the valve and the delivery system, are configured by loading and aligning the valve onto the delivery system during preparation.

According to an advantageous example, a method for delivering a prosthetic aortic valve to an implantation site comprises the following steps:

- determining a planar angle of an aortic arch of a body to the body, defining a plane that aligns perpendicular to the axis that the ascending aorta and native trans-aortic valve sit;

- determining a right coronary angle, $\alpha_r$, relative to the plane around a circumference of an annulus, determining a left coronary angle, $\alpha_l$, relative the plane around the circumference of the annulus;
- determining an implantation angle, $\alpha_i$, corresponding to an angle that a primary commissure of the prosthetic aortic valve is configured to make relative to the reference plane as

$$\alpha_{i-anti-clockwise} = 90^o - \left( \left( \frac{\alpha_r + \alpha_l}{2} \right) - 180^o \right)$$

or $$\alpha_{i-clockwis} = 270^o - \left( \left( \frac{\alpha_r + \alpha_l}{2} \right) - 180° \right);$$

- arranging a rotatable socket configured to rotate relative to an inner shaft and a valve capsule;
- prior to loading the prosthetic valve, rotating the rotatable socket to the implantation angle;
- wherein the socket comprises at least one retention recess positioned on an external surface of the socket;
- the retention recess is configured to engage with a paddle of the prosthetic aortic valve, such that the paddle remains detachably locked in the socket;
- prepping the valve in a loading system and placing the at least one paddle of the prosthetic valve into the at least one retention recess of the socket which is fixed at the implantation angle;
- moving the capsule forward to encapsulate the valve.

The rotating socket may come pre-assembled with the device so all that needs to be done at this step is for the TDS to pull back the capsule to expose the rotating socket. A TDS is the person in charge of setting the valve to the correct relative angle and the MRI or CAT scan technician would be in charge of assessing the anatomical dimensions. According to an advantageous embodiment, the step of rotating the rotatable socket to the implantation angle is relative to a plane of inflexibility of the device and the implantation angle is measured relative to the spine-wire. This is advantageous for systems with one plane of deflection. Preferably the spin-wire is a capsule and/or an inner shaft and/or an outer shaft of the device.

The set implantation angle may stay locked in position during the loading process. The step engaging the retention recess with a paddle of the prosthetic aortic valve, such that the paddle remains detachably locked in the socket is done with the aid of a loading tool. Loading tool keeps the paddles in position until the capsule is advanced over the paddles/sockets and proximal half of the valve.

The prosthetic valve may first be placed in a loading tool which radially compresses it in a way to bring the paddles to the sockets and to help reduce difficulty of advancing capsule over valve.

In other words, a procedure of the this method comprises the following steps:

The patient is assessed using MRI or CAT scan to determine the planar angle of the aortic arch to the body and relative to the trans-aortic valvular plane and the angle that the coronaries make relative to that plane around the circumference of the annulus.

A commissure on the TAVI is aligned with a radiopaque marker on the delivery system capsule so that the physician knows the relative orientation of the encapsulated valve during the procedure.

In-Theatre System Loading Prep:

Prior to loading the implant, the implant retention socket is rotated to the desired orientation relative to the capsule.

The TAVI is loaded with one of its commissures aligned with a radiopaque marker on the delivery system capsule.

The valve is then prepped in the loading system and the paddles of the valve placed on the sockets on the retainer, which is fixed in its new desired rotation.

The capsule is moved forward to capture the Valve (some loading steps omitted here for brevity) until the tip of the capsule mates with the tip of the inner shaft.

Possible Procedure (for systems with one plane of deflection):

Once loaded, the system is brought to the sterile space and is passed over the guidewire and into the patient's femoral artery

The system is then tracked through the iliacs, along the abdominal aorta, around the aortic arch until the capsule is in position across the native annulus.

The capsule, with the primary bending plane now parallel to the plane of the aortic arch is then pulled back, exposing and then deploying the valve in its desired orientation across the native annulus.

According to an advantageous example, the step of rotating the rotatable socket to the implantation angle is relative to a handle and/or a flush port and/or a tip of the delivery catheter and the implantation angle is measured relative to the handle and/or the flush port and/or the tip of the delivery device. This method is advantageous for systems with multiple planes of deflection. Advantageously, the implantation angle is measured relative to a point marking an orientation of a handle flush port or other handle feature, which is kept upright by a physician during the procedure.

Another method for delivering a prosthetic aortic valve to an implantation site comprises the following steps:

- arranging a rotatable socket configured to rotate relative to an axis of the delivery catheter;
- the socket comprises at least one retention recess positioned on an external surface of the socket;
- the retention recess is configured to engage with a paddle of the prosthetic aortic valve, such that the paddle remains detachably locked into the retention recess;
- loading the prosthetic aortic valve;
- upon loading the prosthetic valve and/or upon inserting the prosthetic valve into a patient, as the section of the handle connected to the outer shaft and capsule is torqued by a clinician relative to a section of the handle connected to the inner shaft, the torque is transferred to the capsule;
- through friction, the capsule transfers the torque to the prosthetic valve and subsequently to the socket to which the prosthetic valve is secured by means of the at least one paddle of the valve being engaged with the at least one retention recesses of the socket;
- the socket is free to rotate while the distal tip of the device, the inner shaft components and distal section of the handle remain stationary.

This method is applied to systems with multiple planes of deflection.

In other words, the procedure steps of this method may be:

1. Valve loading is performed as standard or as in the first method.

2. Upon inserting the TAVI into patient, the capsule and valve can be re-oriented by the user by rotation of the handle at any point during tracking from femoral access point to the aortic arch and then into position across the native annulus.

3. Using Echo and Fluoro, the user can determine the position of the coronary artery ostia relative to the implant by assessing the relative orientation of a specific commissure mark on the implant on the prosthetic valve (implant) or other orientation feature. This can be radiopaque to aid visualization.

4. Ascertaining the implant commissure relative positions, the user can then reorient the implant so that the commissures or other implant features are positioned to allow optimal access to coronary artery ostia

5. Once satisfied with the position of the implant, user can then retract the capsule to expose and then deploy the implant in the optimal orientation for commissural alignment.

Advantageously, this method allows selective commissure alignment before and/or during in-vivo positioning/deployment that will allow for PCI access for potential future. This method is preferably applied to omni-directional flexible systems. The independently rotatable retainer is used, to help define the desired orientation of the valve within the body. Along with having the ability to orientate the implant during valve loading, the orientation of the valve can also be adjusted before and during deployment or at any stage while tracking the device through the anatomy.

Advantageously, the capsule is manually rotated relative to the inner shaft upon the clinician rotating a section of the handle attached to the capsule by means of an outer sheath. The implant can freely rotate relative to the inner shaft.

In this method, the optimum implant angular position can be determined relative to some other feature on the delivery device. This may be an existing feature such as a flush port on the actuator/handle or a specifically created feature on the handle/actuator such as a visual and/or tactile mark.

As the implant is in a state of compression within the capsule and is applying a radial outward force on to the inner surface of the capsule, the implant will tend to rotate with the rotation of the capsule. The implant is restrained from axial movement by the implant retention socket. As the socket is free to rotate around the inner shaft, the implant can also freely rotate around the inner shaft as long as there is sufficient clearance between the compressed implant and the inner shaft inside the compressed implant, distal of the rotating socket. This allows the implant to be rotated around its own axis in order to align the implant commissures or other features of the implant to best avoid obstruction of the coronary arteries. This rotation of the capsule and implant around its own axis while the delivery device is in position in the aorta/aortic arch and taking its curvature is possible in a system which has a capsule and outer shaft or other such elements which are free to bend in all directions and do not have a primary or single bend direction. The inner shaft may or may not be flexible in all directions as it does not need to rotate about its own axis in this configuration.

As the inner shaft is not required to rotate during the implant rotation, its bending stiffness, either single plane or other, does not prevent the outer shaft and capsule from being able to rotate around their own axis. A steering system and/or a method of locking the inner shaft in its deflected position, once centered in the native valve, will provide a stable base for the capsule and implant rotation around the assembly axis.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Furthermore, several aspects of the embodiments may form-individually or in different combinations-solutions according to the present invention. The following described embodiments thus can be considered either alone or in an arbitrary combination thereof. Further features and advantages will become apparent from the following more particular description of the various embodiments of the invention, as illustrated in the accompanying drawings, in which like references refer to like elements, and wherein:

**FIG. 1** is a schematic perspective of a delivery device of the present invention;

**FIG. 2A** is a schematic perspective of the coronary ostia relative to native commissures and a valve implant;

**FIG. 2B** is a schematic perspective of a native aortic valve;

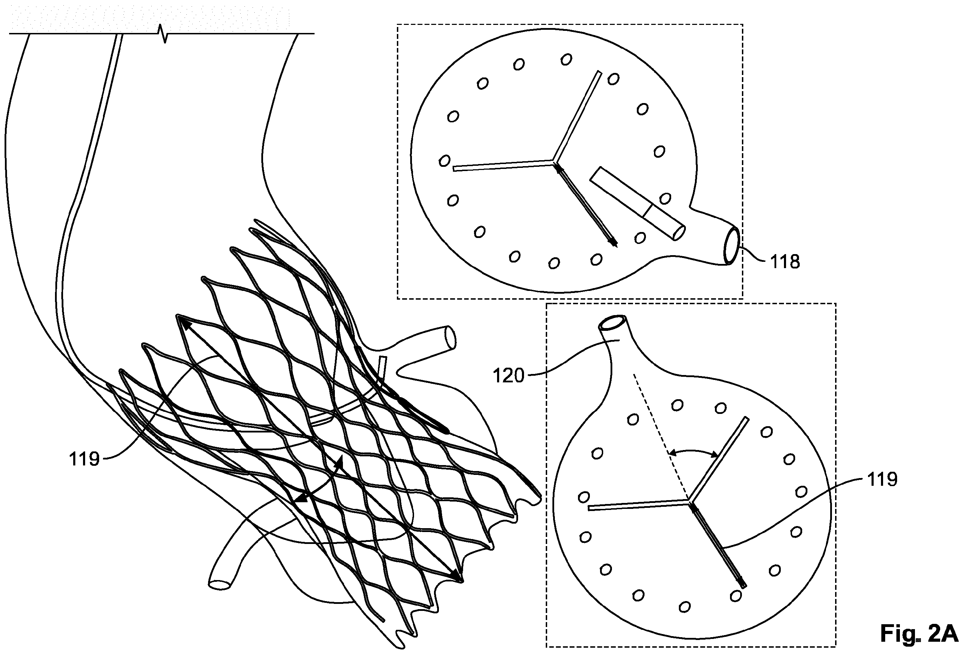

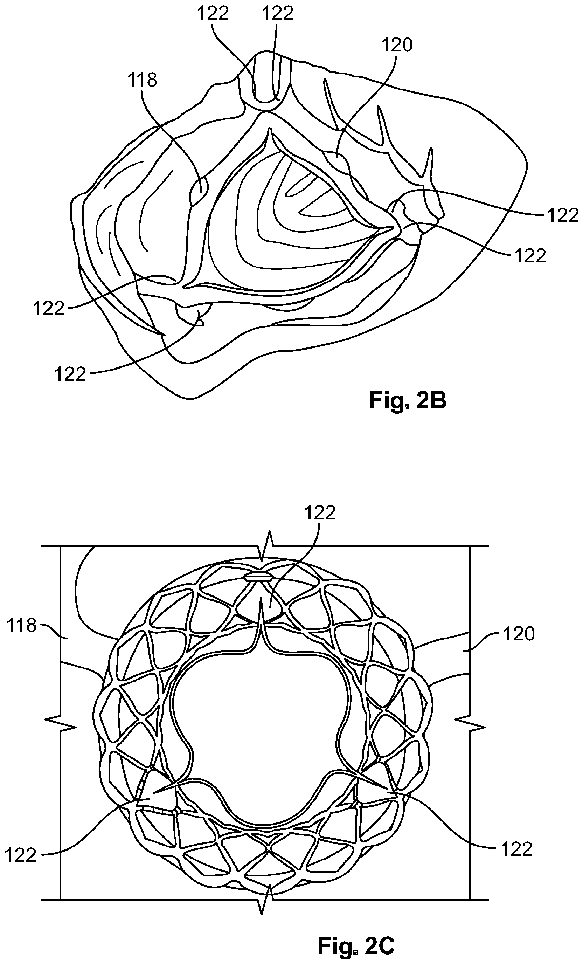

**FIG. 2B** is a detail of the prosthetic aortic valve of figure 2A showing prosthetic and native valve's commissural aliment;

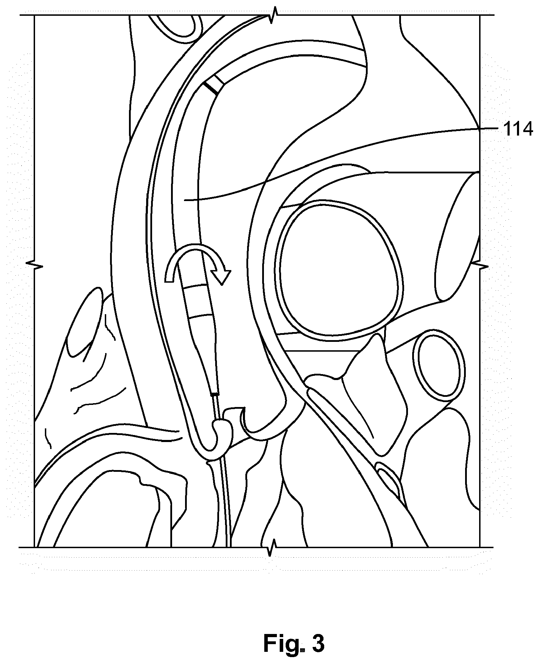

**FIG. 3** shows a representation of the delivery device including the implant containing capsule above the native valve annulus;

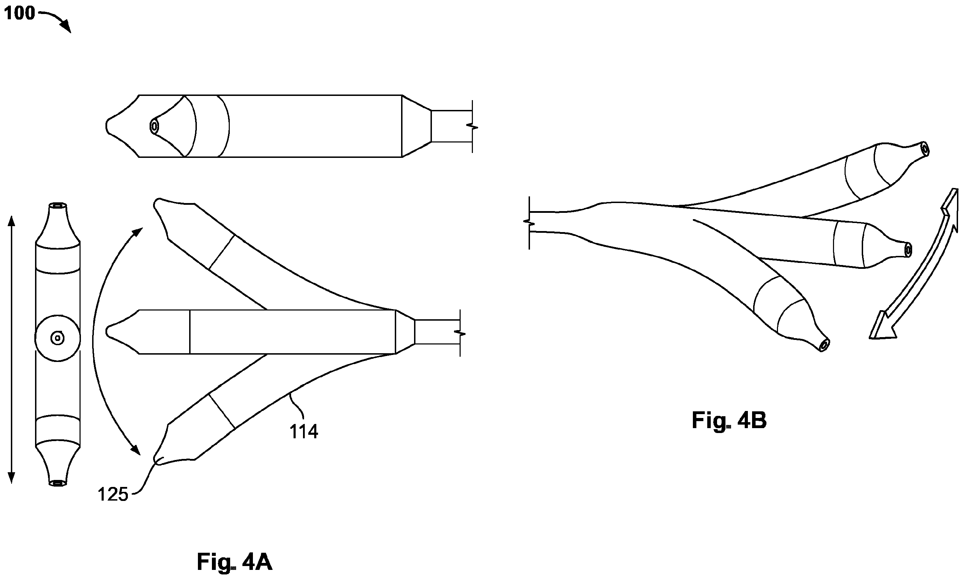

**FIG. 4A** is a schematic representation of an example of planar flexibility of a delivery device of the present invention whereby the device can only principally flex in one plane;

**FIG. 4B** is a schematic representation of another example of planar flexibility of a delivery device of the present invention whereby the device can only principally flex in one plane;

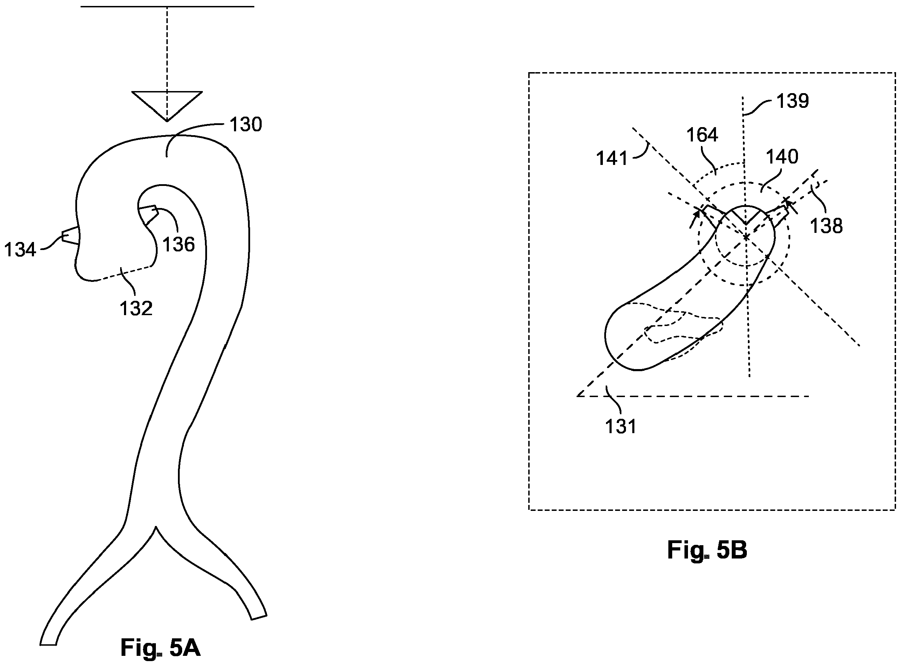

**FIG. 5A and 5B** are schematic representations of an example of aortic anatomy;

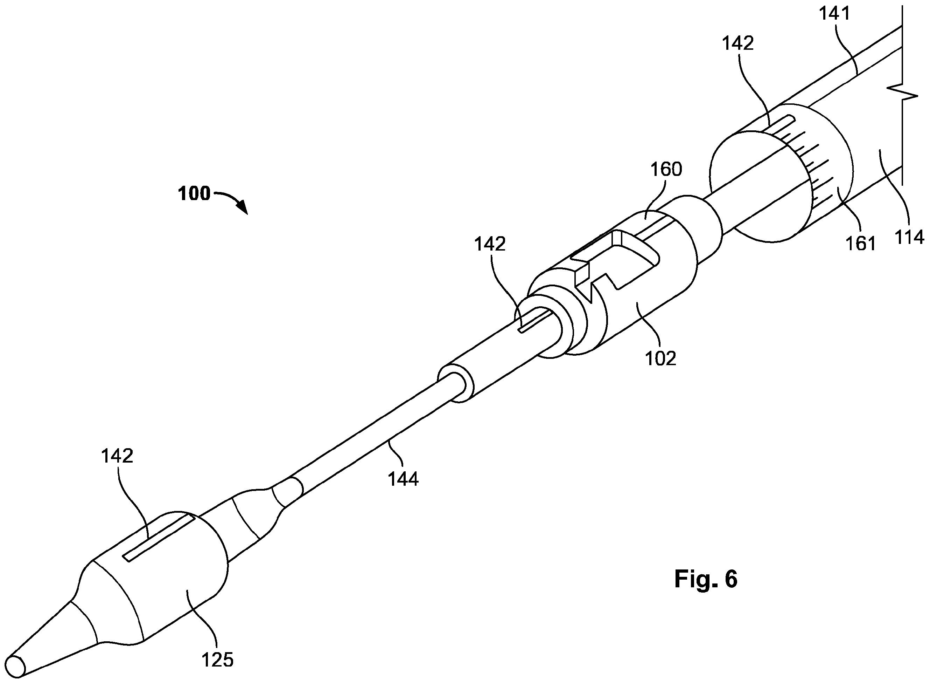

**FIG. 6** is a schematic representation of a device according to an example of the present invention;

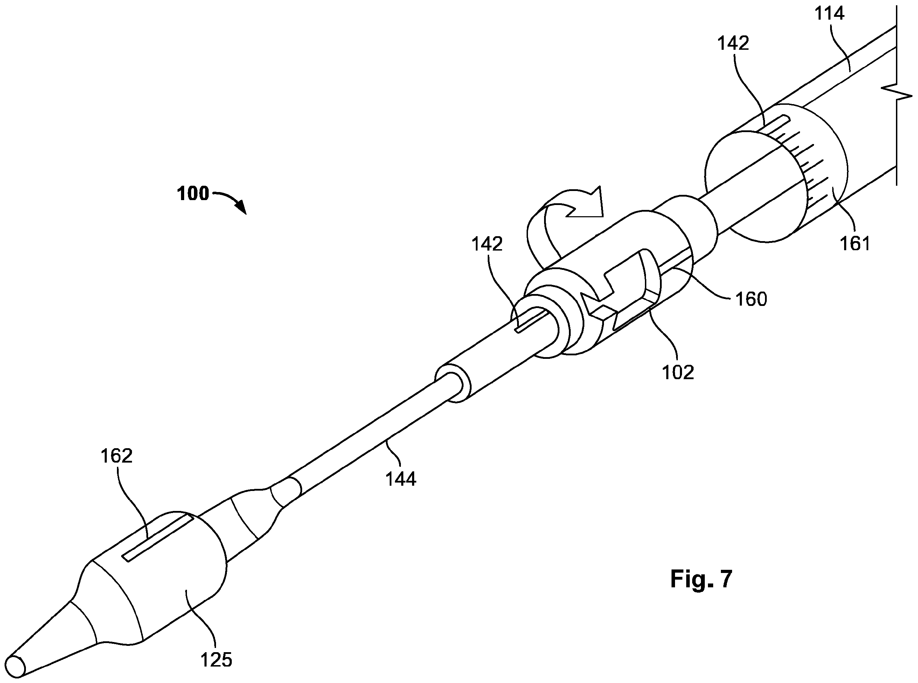

**FIG. 7** is a schematic representation of the device according to an example of the present invention;

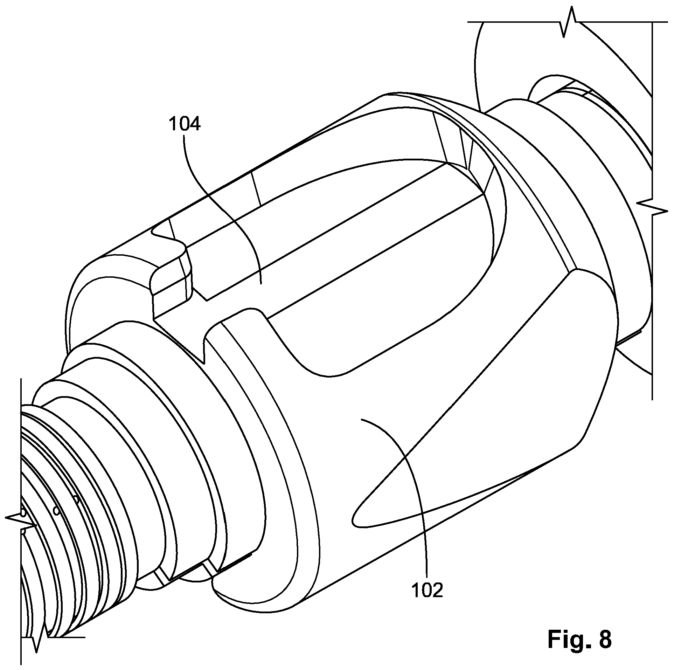

**FIG. 8** is a detail of an example of an implant retention socket of the present invention;

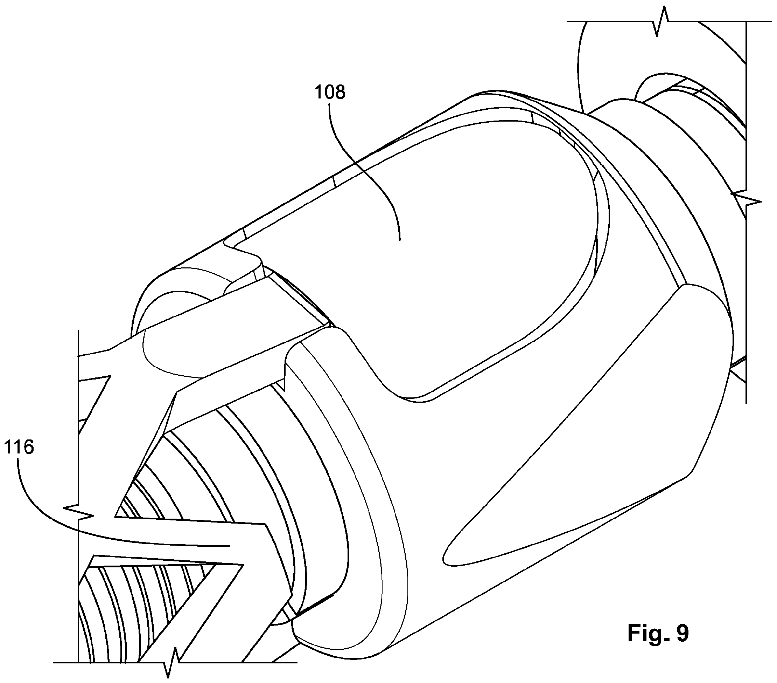

**FIG. 9** is another detail of an example of an implant retention socket of the present invention;

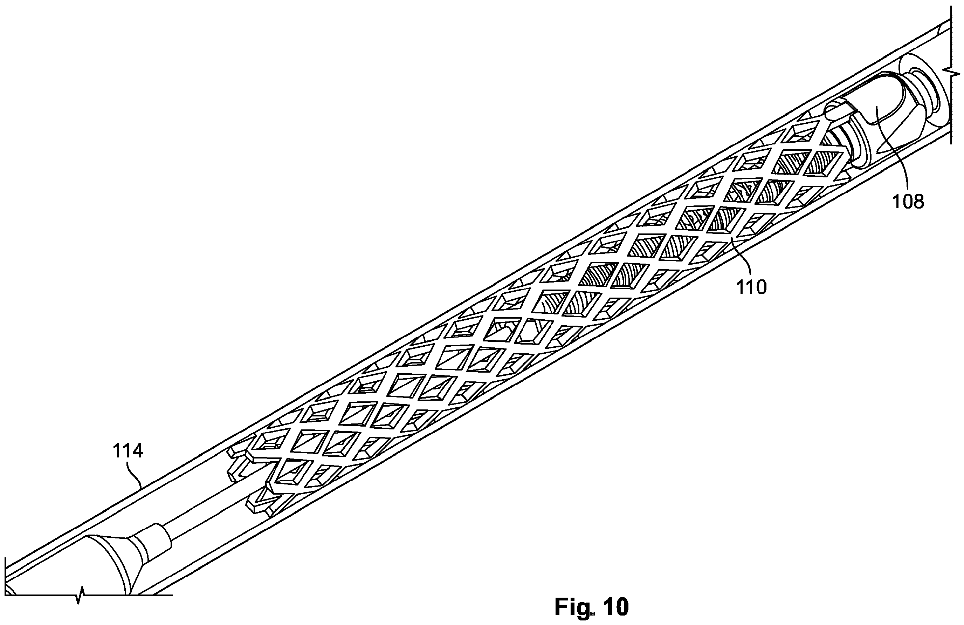

**FIG. 10** is a schematic representation of an example of a delivery device of the invention;

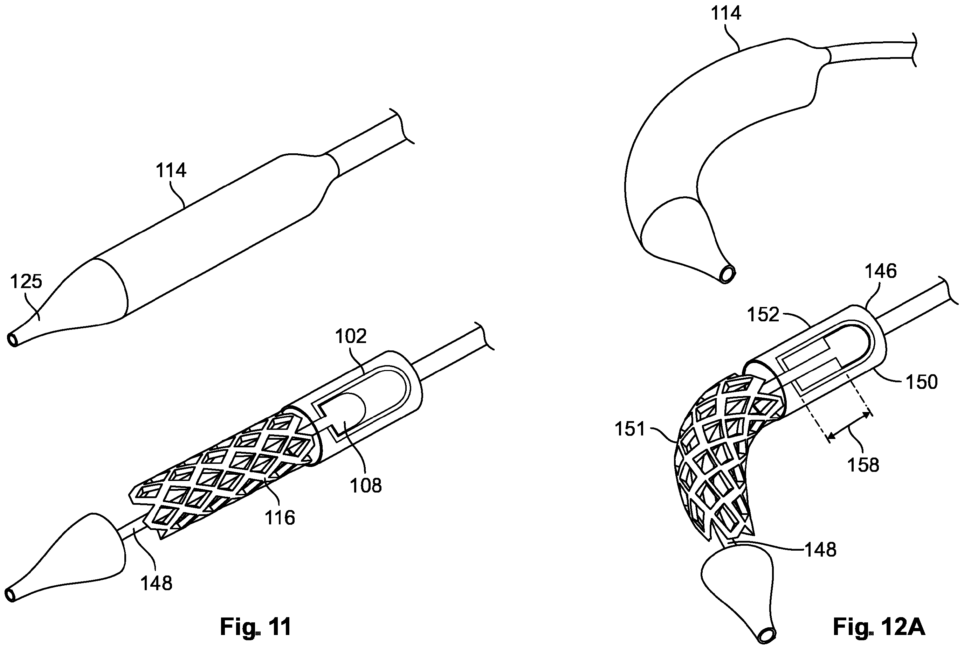

**FIG. 11** is a detail of an example of a delivery device of the present invention ;

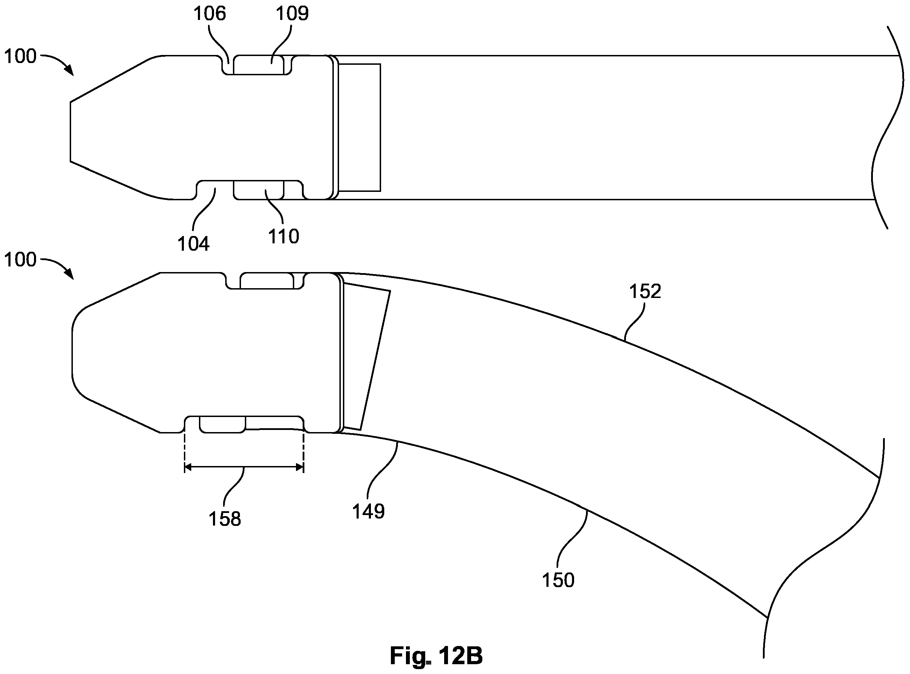

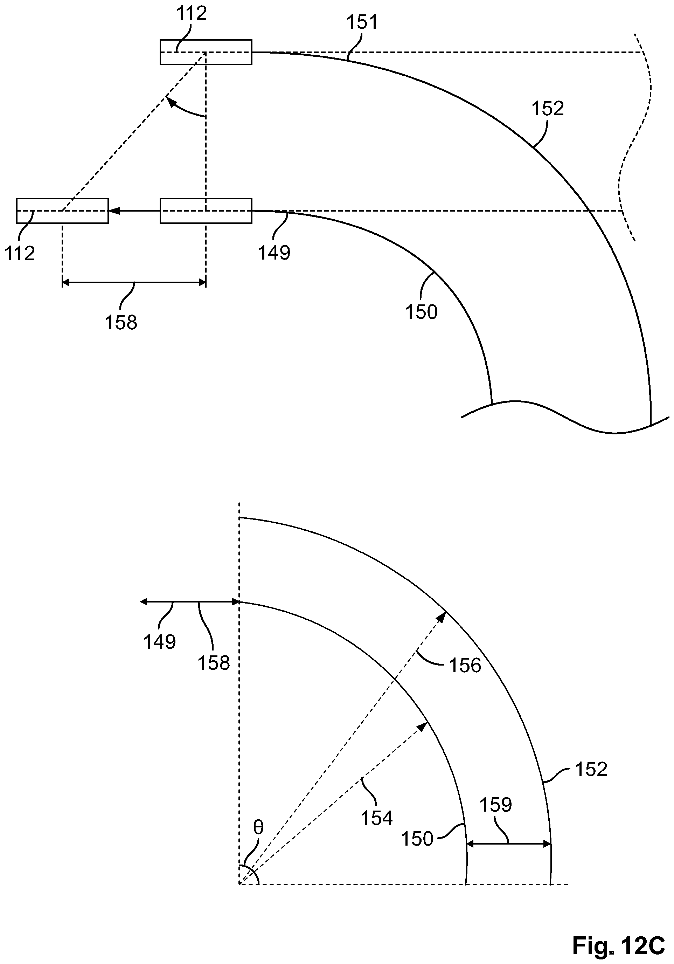

**FIGS. 12A, 12B and 12C** show a detail of an example of a delivery device of the present invention;

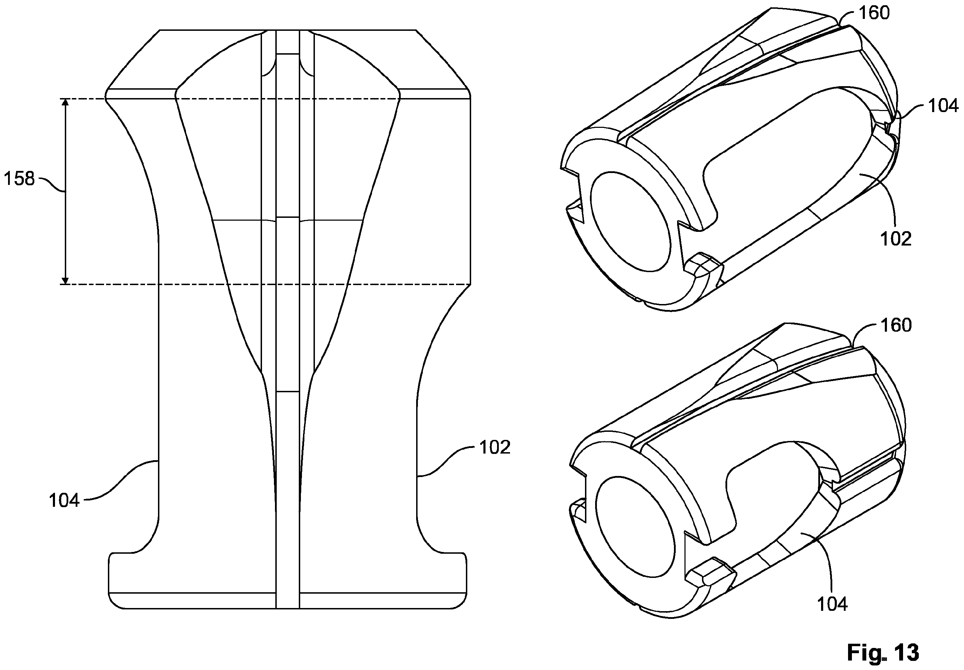

**FIG. 13** is a detail of an example of an implant retention socket of the present invention;

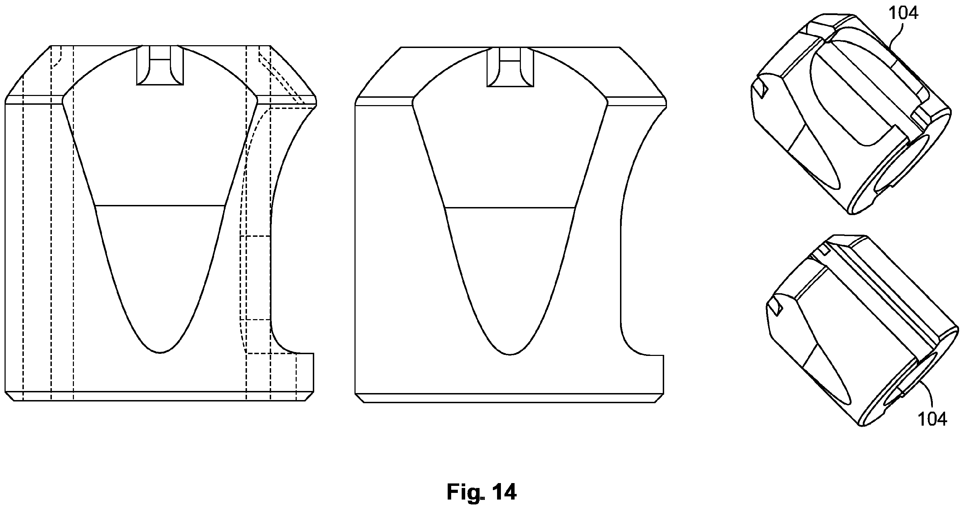

FIG. 14        is a detail of an example of an implant retention socket of the present invention;

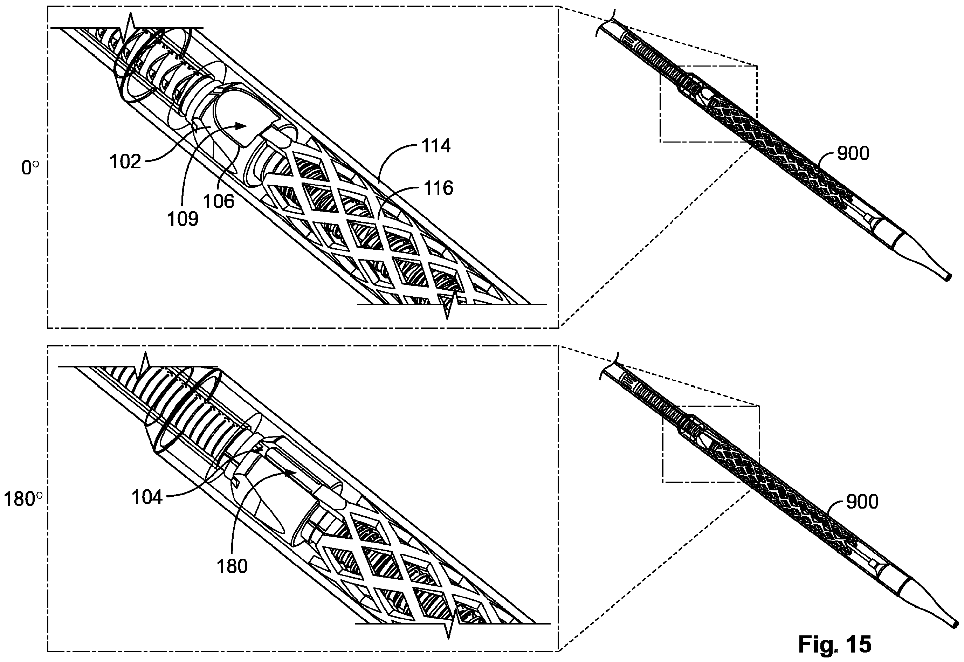

FIG. 15        is an example of a delivery device of the present invention;

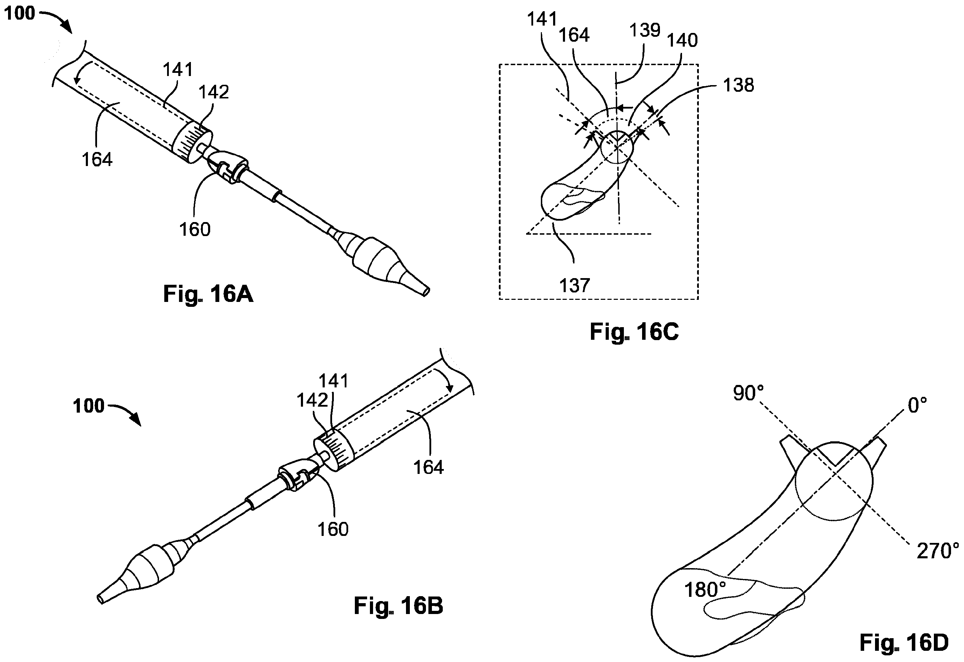

FIG. 16        is a detail of a delivery device according to an example of the present invention;

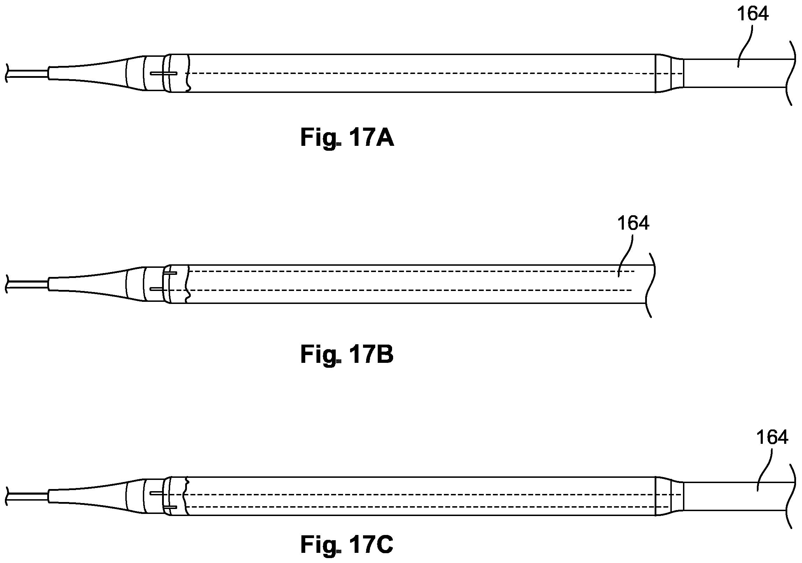

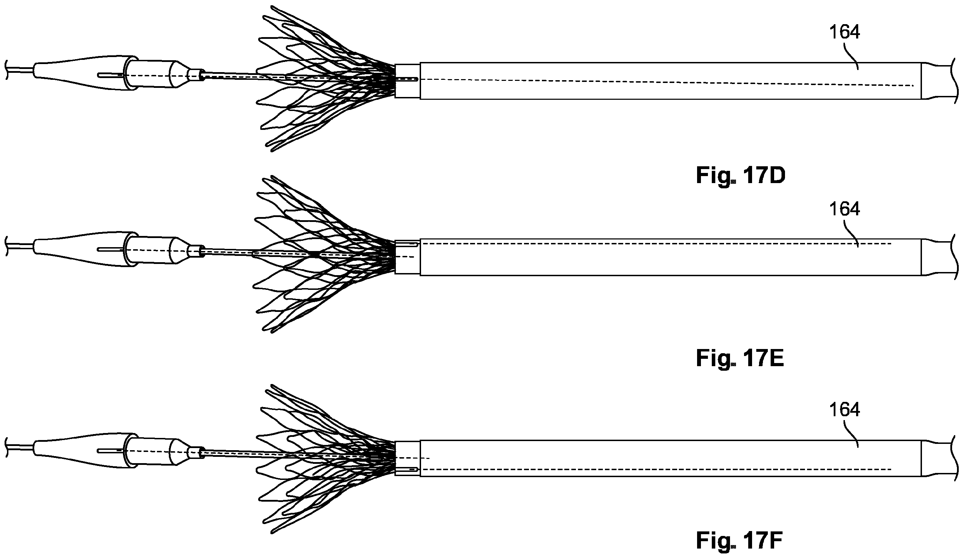

FIG. 17        is a detail of the delivery device according to another example of the present invention;

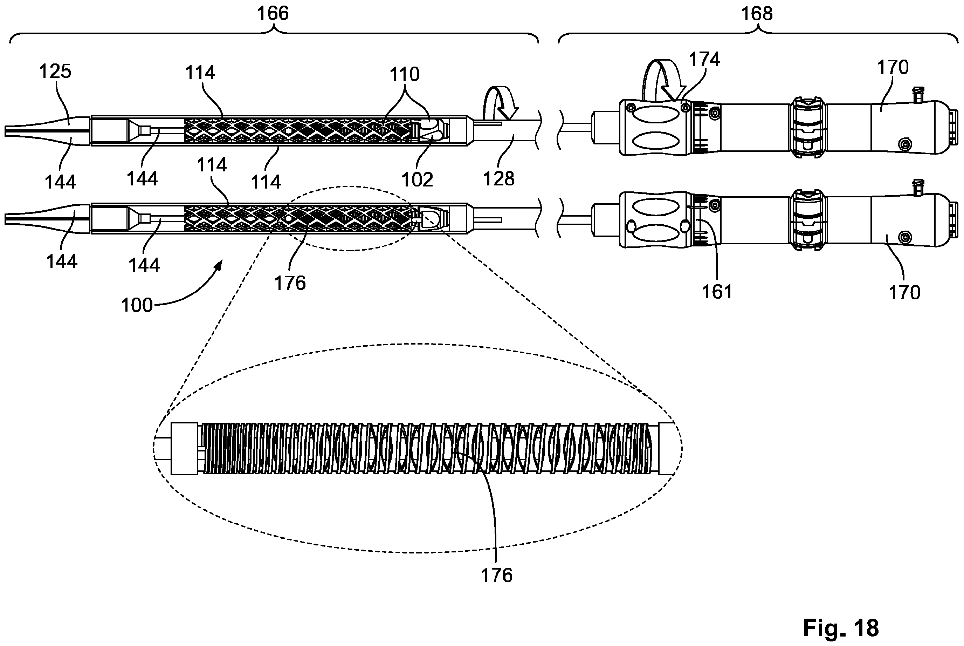

FIG. 18        is a detail of the delivery device according to the embodiment of Fig. 17;

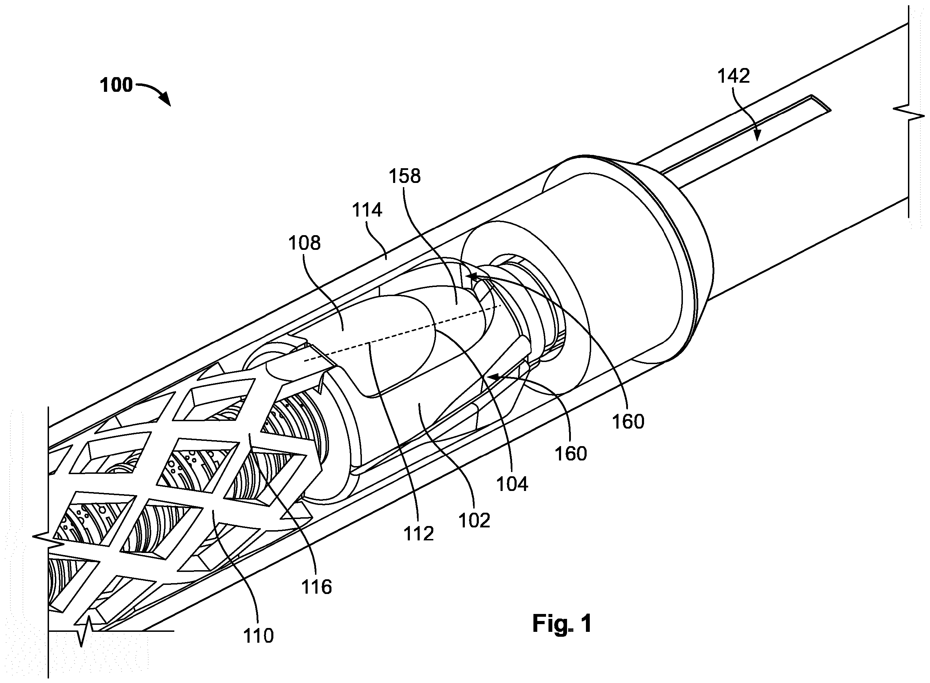

The present invention will now be explained in more detail with reference to the Figures and firstly referring to Fig. 1.

Fig. 1 shows a prosthetic aortic valve delivery device 100 comprising an exemplary socket 102. The socket is an example of one of the possible sockets of the present invention.

The delivery device comprises a proximal end locatable in proximity to a clinician and a distal end locatable distant from the clinician. An axis of the delivery device extends from the proximal end to the distal end. The axis of the delivery device may be referred to as a longitudinal axis or as a central axis.

The exemplarily socket comprises a retention recess 104 positioned on an external surface of the socket for engaging with a paddle 108 of the prosthetic aortic valve 110 such that, upon being engaged, the paddle 108 remains detachably locked into the retention recess 104.

Prior to loading the prosthetic valve, the retention socket 102 can easily be rotated to a desired orientation angle 164 i.e. implantation angle, relative to an axis of the delivery device such as a catheter shaft's plane of inflexibility, a capsule 114 or relative to any stiff axial element of the catheter. The socket can be fixed at the rotation position corresponding to the later implantation angle 164 at which the commissures of the prosthetic valve 110 do not overlap with the native coronary arteries i.e at the rotational position at which the commissures of the prosthetic valve are aligned with the native commissures. In other words, it allows commissural alignment between prosthetic and native aortic valve leaflets.

The prosthetic valve 110 can be prepped in the loading system and at least one paddle 108, 109 of the prosthetic valve can be placed in the retention recess 104 of the socket remaining fixed at the desired rotation. The at least one retention recess 104 of the socket is configured to engage with a paddle of the prosthetic valve such that the recess fits and holds the paddle. Thus, the valve paddle can be seated on the retention recess during loading with a special loading tool.

The figure shows an example of a socket with one retention recess. However the socket may have more than one retention recesses. One retention recess 106 may fit a paddle of the prosthetic aortic valve so that the paddle 109 is restrained axially, while all other recesses 104 allow some axial movement of the paddles 108. This configuration reduces stresses in the struts due to bending of the prosthetic valve (implant) inside the bending capsule. The bending causes the strut lengths and paddles to move axially relative to the socket/recesses so if all recesses constrained axial movement then the stresses in the paddles/struts would be higher than if all but one allowed axial movement as in the present invention.

The socket 102 is independently rotatable to the rest of the system. For example, it is able to rotate relative to the inner shaft, the outer shaft, the capsule 114 and a handle of the delivery catheter.

Prior to loading the prosthetic valve 110, the socket can easily be rotated to a desired orientation angle i.e. implantation angle relative to the capsule or relative to any other stiff axial element of the catheter. The implantation angle is one that allows for alignment i.e. superposition, of the prosthetic valve's commissures with the native commissures of the patient. In other words, implanting the prosthetic valve at the correct implantation angle avoids that the commissures of the prosthetic valve overlap with the left coronary ostium and the right coronary ostium, corresponding the left coronary artery and the right coronary artery.

In other examples the socket may comprise one or more than one retention recesses, wherein at least one retention recess fits a paddle of the prosthetic aortic valve so that the paddle is restrained axially, while all other recesses have a larger length allowing some axial movement of the paddles.

Although not shown in the figure, the retention socket may comprise locking means configured to lock rotation of the socket upon being rotated to an implantation angle.

The figure shows a marking 160 in the retention socket to indicate an orientation of the socket relative to a capsule of the delivery device or relative to a tip or a handle of the delivery device. The socket may comprise an indexing feature around a circumference to index degrees of rotation.

In Fig. 1 the prosthetic valve 110 is prepped in the loading system i.e. it is assembled in the device, and the paddle 108 of the valve is placed in the retention recess of the socket which may be fixed at the implantation angle i.e. at the desired orientation. The capsule 114 is positioned in the device maintaining the prosthetic aortic valve in a collapsed state position between the inner shaft and the valve capsule. To keep the frame 116 of the prosthetic valve from expanding before reaching the target location, the capsule 114 houses and retains the valve 110 as it passes through the patients vasculature. At the target location the capsule 114 is pulled back to allow the valve to expand and deploy.

The delivery device has a longitudinal axis around which the socket is arranged to rotate. The longitudinal axis may be referred to as axis.

The prosthetic valve 110 comprises a frame 116 and a at least one paddle 108. The at least one valve paddle 108 is engaged with the retention recess 104.

Fig. 2A shows the implantation angle optimizing commissure alignment of the prosthetic valve to facilitate PCI access. It shows one commissure plane 119 of the three commissure planes. The commissures of the prosthetic valve are aligned with the native commissures of the patient, avoiding superposition of the prosthetic valve commissures with the left and right coronary ostia.

Fig 2B. shows a schematic representation of a native aortic valve, including the native valve commissures 122, the native left coronary ostium 118 and the native right coronary ostium 120. Ideal implant placement facilitating PCI access. The left and right coronary ostia 118, 120 are seated in the left and right aortic cusp of the native aortic valve.

Fig. 2C shows a schematic view of the prosthetic aortic valve positioned at the implantation angle with its commissures aligned with the native commissures, facilitating PCI access.

Fig. 3 shows the system i.e. catheter being tracked through the iliacs, along the abdominal aorta, around the aortic arch until the capsule is in position across the native annulus. Once in the native annulus, the capsule, with the primary bending plane now parallel to the plane of the aortic arch is then pulled back, exposing and then deploying the valve in its desired orientation across the native annulus.

Figs. 4A and 4B show a delivery catheter 100 with a primary plane of deflection 126. The plane of deflection 126 is the plane in which the catheter can be bent. In other words the plane of deflection is a flexible plane. It shows a spine-wire 124, an outer shaft 128 and the capsule 114. The spin-wire may be referred to as axial stiffness element.

Delivery catheters with a single plane of deflection are not-flexible in-line with a spine-wire 124 plane and are flexible 90 degrees to the spine wire plane. Axial stiffness of the inner and outer sheath components of a delivery catheter may be maximised by addition of stiff axial elements which restrict bending out of the primary bending plane. The position of a stiff axial element defines a spine-wire 124 plane. In this configuration, measuring the implantation angle relative to the spin-wire plane may be advantageous and make the measurement accurate. The figure also shows the capsule 114 of the prosthetic valve, the outer shaft 128 and a tip 125.

Fig. 5A shows an aortic arch 130, an annulus 132, a left coronary artery 134 and a right coronary artery 136. The location of the ostia, the native commissures and the orientation of the ascending aorta and arch can be determined by pre procedural imaging, allowing an optimal rotational alignment of the implant (prosthetic aortic valve) relative to the delivery device to be determined before implantation to allow optimal alignment of implant commissures with the native commissures.

Fig. 5B shows an aortic arch angle 131, in other words, a planar angle 131 of an aortic arch of a body to the body. The body may be the body of a patient. It shows the plane of device inflexibility 141 and an ideal commissure plane 139. It shows the right coronary angle 138, $\alpha_r$, that a right coronary artery of the body makes relative to the reference plane 131 around a circumference of an annulus 132, a left coronary angle 140, $\alpha_l$, that a left coronary artery of the body makes relative to the reference plane 131 around the circumference of the annulus 132. The socket is configured to be rotatable relative to a capsule of the prosthetic aortic valve, an implantation angle corresponding to an angle that a primary commissure of the prosthetic aortic valve is configured to make relative to a reference plane defined by a planar angle of an aortic arch of a body to the body.

The implantation angle may be determined by determining a right coronary angle, $\alpha_r$, that a right coronary artery of the body makes relative to the reference plane around a circumference of an annulus, determining a left coronary angle, $\alpha_l$, that a left coronary artery of the body makes relative to the reference plane around the circumference of the annulus and determining the implantation angle as $\alpha_{i-anti-clockw} = 90^o - \left( \left( \frac{\alpha_r + \alpha_l}{2} \right) - 180^o \right)$ or as $\alpha_{i-clockwis} = 270° - \left( \left( \frac{\alpha_r + \alpha_l}{2} \right) - 180^o \right)$ as explained also in Figs 16A-16D.

The patient may be assessed using MRI or CAT scan to determine the planar angle of the aortic arch to the body and the angle that the coronaries make relative to that plane around the circumference of the annulus (see figures 5A and 5B).

Figs. 6 and 7 show an example of delivery catheter i.e. device, comprising an exemplarily rotating socket 102, a capsule 114, orientation markers 142, an inner shaft 144 and a tip 125.

The socket may comprise a marking i.e. an orientation marking, which for example may be a cut such as a 90° cut as shown in the figure. In this example, the socket comprises a marker to indicate the relative orientation of the rotating socket relative to a plane of inflexibility of the device e.g. relative to the capsule and/or inner shaft and/or outer shaft. However, in the other examples the relative orientation of the socket may be measured relative to the a tip and/or a handle of the delivery catheter.

To facilitate selective orientation of the TAVI within the delivery system, the socket, in other words in other words valve retainer, is made independently rotatable to the rest of the system, rotating relative to the inner shaft, the outer shaft and the handle. This can be seen in Figs. 6 and 7. The capsule 114 can have a marker 142, aligned with the primary bending plane or other feature, that allows the user to determine the valve orientation relative to the primary bending plane or other device feature.

The self expanding TAVI device may have two main shafts, an inner shaft and an outer shaft. The inner shaft may have, at the distal end, the (implant) retention socket comprising one or more recesses shaped to engage with one or more paddles fixed to the implant. The paddles may be called retention features.

As the rotating socket is rotated into the chosen position, both the capsule and inner shaft, in other words inner catheter, may remain fixed in their orientation. The rotating socket can have an indexing feature which allow it to index around the circumference at fixed degrees of rotation. The rotating socket can also have a locking feature which locks rotation once set to the preferred angular orientation. The figure exemplary shows an indexing feature in the capsule.

Fig. 8 shows an example of a socket 102 with a detail of an example of a shape of a retention recess 104. The retention recess is shaped to fit and hold a paddle 108 of the prosthetic aortic valve. The figure shows exemplarily that there may be at least one collar retention for the valve socket but other alternatives are possible. The retainer i.e. socket, may retain the valve by way of one or more collared interactions. Examples of other shapes of retention recesses of sockets are shown in Figs. 14 and 15.

Figures 14 and 15 show that the valve is retained axially by only one collared paddle, while the other paddle has no collar but acts as a guide for movement of the valve and helps prevent relative torsion between the valve and retainer.

Fig. 9 shows an example of a shape of a valve paddle 108 engaged with a retention recess 104. The figure shows the prosthetic valve comprising the valve frame 116 i.e. frame or struts. Valve paddles may be seated on the retainer during loading with a special loading tool.

Fig. 10 shows a capsule 114 preventing radial expansion of a prosthetic aortic valve. The prosthetic valve cannot move axially due to locked paddle. When the capsule 114 moves fully forward to encapsulate the valve, valve paddle is locked into the retainer by the capsule.

A paddles locates the implant in the delivery device, by engaging with the recess of the socket. The figure shows one paddle engaged in a retention recess, however, the socket may have more than one retention recess engaged with other paddles. The paddles constrain the longitudinal movement of the implant relative to the inner shaft and also fix its rotational position with respect to the shaft axis. As shown in the figure, the socket recesses are open on the outside so that the paddle or valve feature can move radially outwards from the socket. The outer shaft may have a hollow cylinder, in other words capsule, at its distal end that constrains the radial movement of the paddles. When the capsule is in a fully forward position, the implant is compressed within the capsule and cannot move relative to the capsule or the inner shaft. Withdrawal of the outer shaft relative to the inner shaft allows the implant to spring out in a controlled manner from the distal end first. The paddles on the proximal end of the implant are constrained from radial movement by the capsule until the distal end of the capsule is withdrawn to a position proximal of the paddles at which point the paddles are free to spring out radially, thereby fully releasing the implant from the delivery device.

Fig. 11 shows in its upper part an example of a delivery catheter with a capsule and in the lower part an example of a delivery catheter without a capsule or, in other words, with a transparent capsule, showing a retention recesses 102 of the socket.

The delivery catheter 100 is shown in a straight position in which the proximal end 146 and a distal end 148 form a 0 degrees angle.

The lower figure shows an example of a paddle 108 of the prosthetic aortic valve engaged such that it remains detachably locked into the retention recess 104. The retention recess 104 has an axis of symmetry 112 along which a length of the paddle 108 configured to engage in that recess 102 extends. The difference between the length of the retention recess and the length of the paddle allows the paddle one degree of freedom of movement along the axis.

Figs. 12A shows an example of a delivery catheter is subjected to a bent in a plane of deflection. Fig. 12A shows an example of a socket with one retention recess. However more retention recesses are possible. The upper part of Fig. 12A shows the catheter with the capsule. The lower part of Fig. 12A shows the catheter without the capsule, showing the valve frame and an example of retention recess and of paddle engaged with the recess.

Figs. 12B and 12C show an example of socket comprising a pair of retention recesses. The upper part of figure 12B shows the catheter in a straight position and the lower one shows a catheter in a bent position. The extra length of one of the recesses on the socket (retainer) acts as a strain relief when the capsule and valve are bent. The extra space on one side allows slight axial displacement for the inside of a bend. If there was no extra space the struts of the valve leading to the paddles would be put under greater stresses.

Figs. 12B and 12C show that when the delivery catheter is subjected to a bend in a plane of deflection containing the symmetry axis 112 of the two retention recesses of the pair, the catheter passes to a bent position in which the proximal and distal ends form an angle $\phi$. In the bent position, the section of the catheter contained in the plane of deflection comprises an inner arc 150 i.e. inner curve and an outer arc 152 i.e. outer curve are concentric to each other.

As an example it is shown that when pulling from a generatrix 149 of the delivery device 100 (or analogously of the prosthetic aortic valve 110), to bend the delivery device in a deflection plane, the device is bent in a deflection plane containing that generatrix 149. Upon undergoing the bent in that plane of deflection, the device passes from an straight position in which a proximal end a distal ends form a 0 degrees angle to a bent position in which the proximal and distal ends form an angle $\phi$. In the bent position, the section of the delivery device contained in the plane of deflection comprises an inner arc 150 and an outer arc 152 concentric with each other.

Fig. 12B shows an exemplary embodiment of the present invention in which the socket comprises two retention recesses.

In this example, the socket comprises a pair of retention recesses 104, 106. The two retention recess are symmetrically positioned on opposite sides of an external surface of the socket 102. Each of the two retention recesses 104, 106 are configured to engage with a paddle 108, 109 of the prosthetic aortic valve 110 such that the paddles 108, 109 remain detachably locked into the retention recesses 104, 106. Each retention recess 104, 106 has an axis of symmetry 112, 113 along which a length of the paddle 108 configured to engage in that recess extends. The length along the axis of the recesses 104 is larger than the length along the axis of the paddle 110 engaged with that recess 104. A length along the axis of the other recess 106 equals the length along the axis of the paddle 109 engaged with that other recess.

This allows the paddle 108 engaged with the recess 104 of larger length to have one degree of freedom of movement along the axis 112, while the paddle 109 engaged with the other recess 106 cannot move axially, upon the delivery device 100, in other words the prosthetic valve, undergoing a bend in a plane of deflection associated to that pair of retention recesses 104, 106, the plane of deflection containing the axes 112, 113 of the two retention recesses of that pair.

Exemplarily, the generatrix from which the device was pulled comprises a bent part i.e. inner arch and a straight part 158. The straight part 158 corresponds to the distance that the generatrix is pulled. The generatrix located symmetrically on the opposite site of the surface of the delivery device comprises only a bent part i.e. outer arch, in other words it has not been displaced, only bent.

In this example, the generatrix from the which the device is bent coincides with the axis of symmetry 112 of the retention recess 104 having a length larger than the length along the axis of the paddle 108 configured to engage with that recess, so that the extra length 158 of the retention recess allows to accommodate for the straight part corresponding to the distance that the generatrix is pulled. The retention recess having an axis coincident with the generatrix placed symmetrically to the pulled generatrix i.e. the retention recess coincident with the outer arch 152, does not have to accommodate any linear movement of the paddle with which it is engaged, thus its length corresponds to the length of that paddle 109. This allows the paddle 108 configured to engage with the retention recess 104 of larger length one degree of freedom of movement along the axis which accommodates for the distance that the delivery device was pulled to cause bending in the plane of deflection containing the axes of the two retention recesses of the pair, while the paddle configured to engage with the other recess cannot move axially.

The explanation referring to bending in a deflection plane may be done with reference to bending of the delivery device or with reference to bending of the prosthetic valve. With reference to the figures the explanation refers to bending of the delivery device, however they analogously apply if bending of the prosthetic valve is used instead.

In this Example, the retaining recesses 104, 106 of the socket 102 fit and hold two paddles 108, 109 of the prosthetic valve 110.

Fig. 12C shows a detail of bending of the prosthetic valve, in other words, a detail of a prosthetic valve subjected to bending. The generatrix 149 from which the prosthetic valve is pulled comprises a bent part 150 i.e. inner arch, and a straight part 158. The straight part 158 corresponds to the distance that the generatrix is pulled. The generatrix 151 located symmetrically on the opposite site of the surface of the prosthetic valve i.e. ourter arch, comprises only a bent part 152 i.e. outer arch, in other words, it has not been displaced, only bent.

Exemplarily, the inner arch 150, $L_{inner}$, may have a length equal to $\phi$ times the inner radius 154, $r_{inner}$, of curvature i.e. $L_{inner} = \phi * r_{inner}$ while the outer arch 152, $L_{outer}$, may have a length equal to $\phi$ times the outer radius 156, $R_{outer}$, of curvature i.e. $L_{outer} = \phi * R_{outer}$. The difference in lengths 158 between the outer 152 and inner 150 archs is then $L_{Outer} - L_{inner} = \phi * (R_{outer} - r_{inner})$. The difference in lengths 158 may be referred to in other words as $\phi * (diameter\ of\ the\ beam)$.

The retention recess 104 of larger axial length, may have a function of bending compensation corresponding to the distance 158 that the generatrix of the prosthetic valve was pulled. The valve paddle 108 positioned in the retention recess 104 associated to the inner arch 150, engaged with the paddle that displaces the pulled distance along the axis 112 can move axially along a retention recess 104 having a length that is longer than the length of the engaged paddle by at least an extra length 158 corresponding to the bending angle times the difference between the outer radius of curvature 156 and inner radius of curvature 154 i.e. $\phi^*(R_{outer} - r_{inner})$. In other words, the extra length 158 of the retention recess engaging the paddle located in the inner arc 150 may correspond to a distance that the paddle has to move to avoid being subjected to stresses when the system undergoes a bent. The length of one of the paddle retention recesses is increased to allow the fitted paddle one degree of freedom of movement while the system undergoes a bend. This individual paddle retention recess is axially elongated so that the valve paddle can move axially while still being restrained radially and circumferentially.

The extra length 158 i.e. the pulled distance or straight distance 158 that the paddle associated to the inner arch 150 is displaced of the retention recess configured to engage the paddle positioned in retention recess associated to the inner curve 150 of the bent plane depends on the angle $\phi$ that the plane is to be bent and on the difference between the outer radius of curvature 156 and inner radius of curvature 154 i.e. $\phi^*(R_{outer} - r_{inner})$. In other words, the bigger the angle that the plane deflects the longer extra distance 158 the retention recess 104 configured to engage with the paddle 108 positioned in the recess associated to the inner arch 150 has to have to avoid stresses in the prosthetic valve. Similarly, the longer the distance between the outer 156 and inner 154 radius of curvature ($R_{outer} - r_{inner}$) i.e. the longer diameter of the prosthetic valve, the longer the extra distance 158 the retention recess 104 configured to engage the paddle 108 placed in the recess associated to the inner arc has to have to accommodate bending while avoiding stresses in the prosthetic valve.

The smaller the distance between the inner 154 and outer 156 radius of curvature i.e. inner and outer archs, or in other words the smaller the diameter of the prosthetic aortic valve or the smaller the bending angle, $\phi$, the less extra distance the retaining of longer length may have relative to the other recess of the pair to accommodate bending.

The retention recess of larger axial length, has a function of bending compensation. To avoid that the prosthetic valve's frame 116 (struts) are subjected to stresses. One of the paddles has one degree of freedom of movement while the system undergoes a bend. This individual paddle retention recess may be axially elongated so that the valve paddle can move axially while still being restrained radially and circumferentially.

Similarly, the longer the distance between the outer 156 and inner 154 radius of curvature (the bigger diameter of the catheter) the longer the extra distance the retention recess configured to engage the paddle positioned in the inner arc of curvature has to have to accommodate bending avoiding stresses in the prosthetic valve.

Similarly, the smaller the distance between the inner and outer radius of curvature (inner and outer archs) or in other words the smaller the diameter of the prosthetic aortic valve, the less extra distance 158 the retaining of longer length may have relative to the other recess of the pair to accommodate bending.

For systems (delivery catheters) with multiple planes of deflection, the socket may comprise more retention recesses associated to each plane of deflection. In an example, for each plane of deflection, a pair of retention recesses having axis of symmetry contained in the plane of deflection is present, analogously to the example of one plane of deflection.

This example illustrates how to solve the issue with bending the capsule and implant and the effect this can have on the relative location of the paddles to the recesses using an example of two paddles. This relative movement is always a problem for any number of paddles except for the situation where only paddle or retention feature is used.

Fig. 13 shows an example of the present invention in which the socket comprises two retention recess 104, 106 are symmetrically positioned on opposite sides of an external surface of the socket 102. Each of the two retention recesses 104, 106 is configured to engage with a paddle 108, 109 of the prosthetic aortic valve 110 such that upon being engaged the paddle 108, 109 remains detachably locked into the retention recess 104, 106. Each retention recess 104, 106 has an axis of symmetry 112 along which a length of the paddle 108, 109 configured to engage in that recess 104, 106 extends. A length along the axis 112 of one of the two recesses 104 is larger than the length along the axis of the paddle 108 configured to engage with that recess 104 and, a length along the axis 113 of the other recess 106 equals the length along the axis of the paddle 109 configured to engage with that other recess 106 so as to allow the paddle 108 configured to engage with the recess of larger length 104 one degree of freedom of movement along the axis 112, while the paddle 109 configured to engage with the other recess 106 cannot move axially, upon the delivery catheter 100 undergoing a bend in a plane of deflection associated to that pair of retention recesses 104, 106 which contains the axes 112, 113 of the two retention recesses of that pair 104,106.

As exemplarily shown in Fig. 13, the length of one of the retention recesses 104 is increased to allow that upon being fitted, the paddle 108 has one degree of freedom of movement while the system undergoes a bend. The retention recess 104 is axially elongated so that the implant paddle 108 can move axially while still being restrained radially and circumferentially. This reduces stress on both paddles when bending applied. The figure shows a socket marking 160 (90° cuts or other types of markings). The marking may mark the relative orientation of the rotating socket 102 to the capsule 114. The socket (valve retainer) may accommodate two or more paddles 108, 109 where one retention recess restrains the paddle axially and the others will allow some axial movement of the paddles.

The figure shows a marker 160. The shaft, the capsule 114 or the tip 125 may also have a marker 162 (shown in Figs 1, 6 and 14)

Figs. 14 shows an example of a socket to compensate for implant bending. Exemplarily, the socket has a retention recess of an elongated shape in which a sliding anchor 180, or in other words sliding anchor paddle or valve sliding anchor, can slide. A sliding anchor (shown in Fig. 15) is an example of a valve paddle of elongated shape configured to slide in the retention recess with which it is engaged. The sliding anchor has freedom of axial movement in the retention recess with which it is engaged which in turn allows some relative movement as the system bends, relieving stress accumulation at the paddles/anchors. This reduces stress on both paddles when bending applied.

Fig. 14 shows 90° cuts or other markings may mark the relative orientation of the rotating socket to the capsule. The socket may accommodate one or more paddles where one retention recess may restrain a paddle axially and the other retention recesses may allow some axial movement of the paddles.

Fig. 15 shows a view of an exemplarily socket and valve paddle assembled in a delivery device. The upper device shown in Fig. 15 shows an example of a paddle 109 and a socket with a retention recess 106 at 0° rotation.

The lower device shown in Fig. 15 is another example of a device, comprising a socket 102 with a retention recess and a paddle 180 assembled in the device. The paddle 180 is an sliding anchor configured to slide in the socket with which it is engaged. The lower device shows 180° rotation i.e.each image looks at an opposite side of the system. The sliding anchor 180 may be referred as valve sliding anchor or sliding anchor paddle. Exemplarily the sliding anchor 180 has an elongated shape of constant width.

Fig. 15 also shows the prosthetic valve with the frame 116 and paddles 180 or 109.

Fig. 16A shows possible markings appropriate for systems with one plane of deflection. It shows a marking 160 in the socket 102 and a marking 142 in the capsule 114, indicating the implantation angle 164 that the socket 102 (valve retainer) makes in relation to a device plane of inflexibility 141 or in other words spin wire . A spine wire may be called an axial stiffness element. It shows an implantation angle 141 i.e. angle of the socket relative to the plane of inflexibility, measured counter clock wise

$$\alpha_{i-anti-clock} = 90^o - \left( \left( \frac{\alpha_r + \alpha_l}{2} \right) - 180^o \right)$$

Fig. 16B shows the same references as Fig. 16A but the implantation angle 141 is shown as measured clock-wise which corresponds to an implantation angle $\alpha_{i-clockwise}$ = 270° - $\left( \left( \frac{\alpha_r + \alpha_l}{2} \right) - 180^o \right)$ .

Fig. 16C shows the same references as fig. 5B. Figs 16 C and 16D show that all angles are measured as per the aortic arch plane and the plane of inflexibility.

In systems with multiple planes of deflection the implant angle may be measured relative to a handle flush port or other handle feature rather than a stiff axial element as is the case with single plane deflection devices. As in systems with one plane of deflection, the patient may be assessed using MRI or CAT scan to determine the planar angle of the aortic arch to the body and the angle that the coronaries make relative to that plane around the circumference of the annulus. The additions of both the angles on one side (acute side), divided by two, is the desired implantation angle that the primary TAV implant commissure will make to that plane.

Fig. 17 shows a method according to a another embodiment, of achieving selective commissure alignment before and/or during in-vivo positioning/deployment that will allow for PCI access for potential future procedures.

Fig. 17A shows a case of no rotation, Fig. 17B shows a case of anti-clockwise rotation and Fig. 17C shows a case of clockwise rotation before in-vivo positioning/deployment of the prosthetic aortic valve. Fig. 17D shows a case of no rotation, Fig. 17E shows a case of anti-clockwise rotation and Fig. 17F shows a case of clockwise rotation during in-vivo positioning/deployment of the prosthetic aortic valve.

The method disclosed in figure 17 is similar to the previous method in which the prosthetic aortic valve is rotated before it is deployed, applying to omni-directionally flexible systems, an independently rotatable retainer is used, to help define the desired orientation of the valve withing the body.

Along with having the ability to orientate the implant during valve loading, the orientation of the valve can also be adjusted before and during deployment or at any stage while tracking the device through the anatomy. To achieve this, the device is configured so that the capsule, and the outer shaft connected to the capsule, can be manually rotated relative to the inner shaft by means of rotating one part of the handle. As the implant is in a state of compression within the capsule and is applying a radial outward force on the capsule, the implant will tend to rotate with the applied rotation of the capsule. The implant is restrained from axial movement by the retainer. As the retainer is free to rotate around the inner shaft, the implant can also freely rotate around the inner shaft. This allows the implant to be rotated around its own axis in order to align the implant commissures or other features of the implant to best avoid obstruction of the coronary arteries. This rotation of the capsule and implant around its own axis while the delivery device is in place in the aorta/aortic arch and taking its curvature is only possible in a system which has a capsule and outer shaft or other such elements which are free to bend in all directions and do not have a primary or single bend direction.

The inner shaft and other elements can be designed to have a single plane of bending or multiple planes. The inner shaft is not required to rotate during the implant rotation, so its bending stiffness does not prevent the outer shaft and capsule from being able to rotate around their own axis. A steering system and/or a method of locking the inner shaft in its deflected position centred in the native valve provides a stable base for capsule and implant rotation around the assembly axis.

To facilitate selective orientation of the TAVI within the delivery system, the socket (valve socket) is made independently freely rotatable relative to the rest of the system (the fixed orientation inner and handle and defined rotation of the outer). This can be seen in the below figure. The capsule and outer shaft are required to significantly flexible in all directions with a high level of torquability relative to the inner so that, as the capsule/outer is rotated in tortuous anatomy, it keeps the bend it makes within the anatomy.

The steps may be the following:

"In-Theatre" system use:

- The valve loading is performed as this is done according to well-known standard procedures. Steps omitted here for brevity.
- Upon inserting catheter into patient, the capsule and valve can be re-oriented by the user at the handle at any point during tracking from femoral access point to the aortic arch and then into position across the native annulus.
- Using Echo and Fluoro together, the user can determine the position of the coronaries relative to the valve by assessing the relative orientation of the specific commissure mark on the valve.
- Ascertaining the valve commissure relative positions, the user can then reorient the valve so that the commissures or other implant features are positioned to allow optimal access to coronary artery, ostia etc
- Once satisfied with the position of the valve, the user can then pull back the capsule to expose and then deploy the valve.

Fig. 18 shows an example of a delivery catheter according to another example. The delivery catheter has full system rotational function. It shows a distal device capsule assembly 166 and a proximal device handle assembly 168. A proximal section of handle 170 may remain stationary. Rotating mid-section of handle 174 relative to proximal section of handle 170 may cause rotation of the outer shaft and the capsule. The degree of rotation may be measured by a relative gauge 161. Proximal outer shaft 128 rotates the capsule 114. The capsule 114 rotates the socket 102 and the prosthetic valve 110. A floating coil 176 may eliminate friction between valve and inner shaft by passively rotating with valve. The inner shaft 144 and tip 125 may remain stationary. The figure also shows a detail of the floating coil 176.

When the distal section of the handle 174 is rotated relative to the proximal section of the handle, the torque is transferred to the outer shaft and to the capsule thus being also rotated. Through friction, the capsule 114 then transfers the torque to the prosthetic valve 110 and subsequently to the socket 102 to which is secured to by means of the at least one paddle 108, 109 being engaged with the at least one retention recess of the socket. The socket 102 is free to rotate around the inner shaft 144 axis. During this process, the distal tip 125 of the device, the inner shaft 144 components and the proximal section of the handle remain stationary. The proximal section of the handle is the section with the Luer flush port pointing up at the back. The Luer flush port is a connection on the handle that a syringe can be attached to for flushing the inner lumen with saline and remove emboli. The floating coil 176, having little friction to the assembly it covers, rotates freely with the prosthetic valve 110, acting as a buffer between the valve 110 and the subassembly underneath.

The inner shaft is what sits on the guidewire and supports the retainer and valve axially.

In the figure, the shaft size in the image with the handle and the shaft size in the image with the capsule are in different scales so as to avoid that one is too big relative to the other and to facilitate seeing the small details.

**REFERENCE NUMERALS**

| Reference Numeral | Description |
| --- | --- |
| 100 | Delivery device |
| 102 | Rotating implant retention socket |
| 104 | Retention recess |
| 106 | Retention recess |
| 108 | Prosthetic aortic valve paddle |
| 109 | Prosthetic aortic valve paddle |
| 110 | Prosthetic aortic valve |

(continued)

| Reference Numeral | Description |
| --- | --- |
| 112 | Axis of retention recess of larger length of the pair |
| 113 | Axis of the other retention recess of the pair |
| 114 | Capsule |
| 116 | Prosthetic aortic valve frame |
| 118 | Native left coronary ostium |
| 119 | Native commissure plane |
| 120 | Native right coronary ostium |
| 122 | Native valve commissures 122 |
| 124 | Spin-wire |
| 125 | Tip |
| 126 | Plane of deflection (flexible plane) |
| 128 | Outer shaft |
| 130 | Aortic arch |
| 131 | Reference plane 131 |
| 132 | Circumference of an annulus |
| 134 | Left coronary artery |
| 136 | Right coronary artery |
| 138 | Right coronary angle 138 |
| 140 | Left coronary angle 138 |
| 142 | Marking (capsule marking, inner shaft marking, outer shaft marking) |
| 144 | Inner shaft |
| 146 | Proximal end of delivery catheter |
| 148 | Distal end of delivery catheter |
| 150 | Inner arc (inner curve) |
| 152 | Outer arch (outer curve) |
| 154 | Inner radius |
| 156 | Outer radius |
| 158 | Difference in lengths 158 between the outer and iner archs |
| 160 | Socket marking |
| 162 | Tip marker |
| 164 | Implantation angle |
| 166 | Distal capsule assembly |
| 168 | Proximal handle assembly |
| 170 | Proximal section of handle |
| 172 | Relative gauge |
| 174 | Rotating mid-section of handle |
| 176 | Floating coil |
| 180 | Sliding anchor |

**Claims**

1. A retention socket (102) for retaining a prosthetic aortic valve in a delivery device (100), the retention socket being configured to be rotatable relative to a longitudinal axis of the delivery device (100), the retention socket comprising: at least one retention recess (104, 106) positioned on an external surface of the socket (102) for engaging with a paddle or other retention feature (108, 109) of the prosthetic aortic valve (110).

2. The retention socket according to claim 1, wherein at least one retention recess (104) fits a paddle (108) of the prosthetic aortic valve (110), so that the paddle (108) is restrained axially, while all other recesses allow axial movement of the paddles.

3. The retention socket (102) according to any of the claims 1-2, further comprising locking means configured to lock a rotation of the socket (102) upon being rotated to an implantation angle (164).

4. The retention socket (102) according to any of claims 1-3, further comprising at least a marking (160) to indicate a rotation of the socket relative to a plane of a capsule of the delivery device and/or relative to a tip (125) or a handle (168) of the delivery device.

5. The retention socket (102) according to claim 4, wherein the plane of the capsule is a plane of anisotropic stiffness of the capsule.

6. The retention socket (102) according to any of the preceding claims, further comprising an indexing feature (161) around a circumference to index degrees of rotation.

7. The retention socket according to any of the preceding claims, comprising at least a pair of retention recesses (104, 106), for each pair:

   the two retention recess (104, 106) are symmetrically positioned on opposite sides of an external surface of the socket (102);
   each of the two retention recesses (104, 106) is configured to engage with a paddle (108, 109) of the prosthetic aortic valve (110) such that upon being engaged the paddle (108, 109) remains detachably locked into the retention recess (104, 106);
   each retention recess (104, 106) has an axis of symmetry (112) along which a length of the paddle (108, 109) configured to engage with that recess (104, 106) extends;
   a length along the axis (112) of one of the two recesses (104) is larger than the length along the axis of the paddle (108) configured to engage with that recess (104) and, a length along the axis (113) of the other recess (106) equals the length along the axis of the paddle (109) configured to engage with that other recess (106) so as to allow the paddle (108) configured to engage with the recess of larger length (104) one degree of freedom of movement along the axis (112), while the paddle (109) configured to engage with the other recess (106) cannot move axially, upon the delivery device (100) undergoing a bend in a plane of deflection associated to that pair of retention recesses (104, 106) which contains the axes (112, 113) of the two retention recesses of that pair (104,106).

8. A device (100) for delivering a prosthetic aortic valve into an implantation site, the device (100) having a proximal end locatable in proximity to a clinician and a distal end locatable distant from the clinician, the device (100) comprising a retention socket (102) according to any of claims 1-7.

9. The device (100) according to claim 8, further comprising the prosthetic aortic valve comprising at least a first paddle connected to a frame, the at least first paddle being engaged with the at least one retention recess.

10. The device (100) according to claim 9, wherein the at least first paddle (109) fits a retention recess (104) of the socket so that the paddle is restrained axially;
    the device further comprising at least one second paddle (108) that it is allowed axial movement.

11. The device (100) according to any of claims 7-10, having one or multiple markings, each marking aligned with a corresponding plane of deflection or inflexibility of the delivery device.

12. The device (100) according to any of claims 7-11, further comprising:

a handle connected to the capsule (114) such that when a torque is applied to a section of a handle (174) attached to an outer shaft and to a capsule relative to a section of the handle attached to an inner shaft, by the clinician, the torque is transferred to the capsule;

wherein the capsule (114) is connected to the prosthetic valve by an outward force exerted by the prosthetic valve (110) on the capsule (114) such that the torque is transferred through friction from the capsule to the prosthetic valve and to the socket (102) engaged with the paddles (108, 109) of the prosthetic valve;

the socket is configured to freely rotate relative to the inner shaft.

13. The device (100) according to any of claims 7-12, further comprising at least one free floating coil (176) located between the inner shaft component and the implant, positioned on an inner shaft component distal of the implant retention socket with a space to the inner sheath component, allowing the implant to rotate freely around the inner sheath components upon the capsule and implant being rotated relative to the inner sheath.

14. The device (100) according to any of claims 7-13, wherein at least one of the valve paddles (108, 109) comprises a sliding anchor configured to slide in the retention recess with which it is configured to engage.

15. Method of configuring a device for selective prosthetic aortic valve orientation, comprising the following steps:

determining a planar angle of an aortic arch of a body, defining a plane;

determining a right coronary angle, $\alpha_r$, relative to the plane around a circumference of an annulus, determining a left coronary angle, $\alpha_l$, relative the plane around the circumference of the annulus;

determining an implantation angle, $\alpha_i$, corresponding to an angle that a primary commissure of the prosthetic aortic valve is configured to make relative to the reference plane as

$$\alpha_{i-anti-clockwise} = 90^o - \left(\left(\frac{\alpha_r+\alpha_l}{2}\right) - 180^o\right) \quad \text{or} \quad \alpha_{i-clockwis} = 270^o - \left(\left(\frac{\alpha_r+\alpha_l}{2}\right) - 180°\right);$$

arranging a rotatable socket configured to rotate relative to an axis of the delivery device and rotating the socket to the implantation angle relative to a plane of a capsule, preferably indicated by markings relative to a tip or a handle of the delivery device;

wherein the socket comprises at least one retention recess configured to engage with a paddle of the prosthetic aortic valve, such that that paddle remains detachably locked into the retention recess;

placing at least one paddle of the prosthetic valve in the at least one retention recess of the socket which is fixed at a rotation corresponding to the implantation angle;

moving the capsule forward to encapsulate the valve.

100

142

158

114

108

160

160

104

112

102

116

110

**Fig. 1**

EP 4 309 625 A1

Fig. 2A

23

**Fig. 2B**

**Fig. 2C**

**Fig. 3**

**Fig. 4A**

**Fig. 4B**

EP 4 309 625 A1

Fig. 5B

Fig. 5A

27

**Fig. 6**

EP 4 309 625 A1

**Fig. 7**

EP 4 309 625 A1

Fig. 8

102

104

EP 4 309 625 A1

**Fig. 9**

Fig. 10

**Fig. 11**

**Fig. 12A**

EP 4 309 625 A1

**Fig. 12B**

EP 4 309 625 A1

**Fig. 12C**

**Fig. 13**

**Fig. 14**

Fig. 15

Fig. 16D

Fig. 16C

Fig. 16B

Fig. 16A

**Fig. 17A**

164

**Fig. 17B**

164

**Fig. 17C**

164

Fig. 17D

Fig. 17E

Fig. 17F

Fig. 18

EP 4 309 625 A1

Europäisches
Patentamt

European
Patent Office

Office européen
des brevets

# EUROPEAN SEARCH REPORT

Application Number

EP 22 18 5918

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/263772 A1 (KLIMA DANIEL J [US] ET AL) 20 September 2018 (2018-09-20) * paragraphs [0032] – [0050]; figures 2-7B * | 1-15 | INV. A61F2/24 |
| X | WO 2012/009006 A1 (ST JUDE MEDICAL [US]; WANG HUISUN [US] ET AL.) 19 January 2012 (2012-01-19) * paragraphs [0073] – [0083]; figures 6A-6C * | 1-15 | |
| X | US 2015/025622 A1 (CREAVEN MARIAN [IE] ET AL) 22 January 2015 (2015-01-22) * paragraphs [0031] – [0046]; figures 1,2,4 * | 1-15 | |

TECHNICAL FIELDS SEARCHED (IPC)

A61F

The present search report has been drawn up for all claims

| Place of search | Date of completion of the search | Examiner |
|---|---|---|
| The Hague | 10 December 2022 | Chevalot, Nicolas |

EPO FORM 1503 03.82 (P04C01)

## ANNEX TO THE EUROPEAN SEARCH REPORT
## ON EUROPEAN PATENT APPLICATION NO.

EP 22 18 5918

This annex lists the patent family members relating to the patent documents cited in the above-mentioned European search report.
The members are as contained in the European Patent Office EDP file on
The European Patent Office is in no way liable for these particulars which are merely given for the purpose of information.

10-12-2022

| Patent document cited in search report | | Publication date | Patent family member(s) | | Publication date |
|---|---|---|---|---|---|
| US 2018263772 | A1 | 20-09-2018 | EP 3595586 | A1 | 22-01-2020 |
| | | | US 2018263772 | A1 | 20-09-2018 |
| | | | WO 2018170198 | A1 | 20-09-2018 |
| WO 2012009006 | A1 | 19-01-2012 | AU 2011279727 | A1 | 07-02-2013 |
| | | | BR 112013001024 | A2 | 24-05-2016 |
| | | | CR 20130017 | A | 25-02-2013 |
| | | | EP 2593048 | A1 | 22-05-2013 |
| | | | ES 2478515 | T3 | 22-07-2014 |
| | | | JP 5906239 | B2 | 20-04-2016 |
| | | | JP 2013534452 | A | 05-09-2013 |
| | | | US 2012078350 | A1 | 29-03-2012 |
| | | | WO 2012009006 | A1 | 19-01-2012 |
| US 2015025622 | A1 | 22-01-2015 | US 2015025622 | A1 | 22-01-2015 |
| | | | US 2017172740 | A1 | 22-06-2017 |

EPO FORM P0459

For more details about this annex : see Official Journal of the European Patent Office, No. 12/82